# EUROPEAN PATENT SPECIFICATION

(11) **EP 3 761 975 B1**
(45) Date of publication and mention of the grant of the patent: **28.10.2026**
(21) Application number: 19711657.7
(22) Date of filing: 05.03.2019
(51) Int. Cl.: A61K 31/4025, A61K 9/00, A61K 31/4155, A61K 31/4178, A61K 31/4192, A61K 31/4196, A61K 31/422, A61K 31/4245, A61K 31/425, A61P 11/00, A61P 13/00, A61P 43/00

(54) **CYANOPYRROLIDINES AS USP30 INHIBITORS AND FIBROSIS TREATMENT**
CYANOPYRROLIDINE ALS USP30-INHIBITOREN UND FIBROSEBEHANDLUNG
CYANOPYRROLIDINES EN TANT QU'INHIBITEURS DE L'USP30 ET TRAITEMENT DE FIBROSE

(30) Priority: 06.03.2018 GB 201803568
(43) Date of publication of application: 13.01.2021
(73) Proprietor: Mission Therapeutics Limited, Cambridge CB22 3AT (GB)
(72) Inventor: THOMPSON, Paul William, Cambridge CB22 3AT (GB); PHELAN, Anne, Cambridge CB22 3AT (GB); STOCKLEY, Martin Lee, Cambridge CB22 3AT (GB); KEMP, Mark Ian, Cambridge CB22 3AT (GB)
(74) Representative: Bridle Intellectual Property
(86) International application number: PCT/GB2019/050608
(87) International publication number: WO 2019/171042

(56) References cited:
- WO-A1-2015/183987
- WO-A1-2017/103614
- WO-A1-2017/163078
- MORA ANA L. ET AL: "Mitochondria in the spotlight of aging and idiopathic pulmonary fibrosis", THE JOURNAL OF CLINICAL INVESTIGATION, vol. 127, no. 2, 1 February 2017 (2017-02-01), GB, pages 405 - 414, XP055952967, ISSN: 0021-9738, DOI: 10.1172/JCI87440
- ANONYMOUS: "USP30 Inhibitor programs - Mission Therapeutics", 3 August 2017 (2017-08-03), pages 1 - 3, XP093195084, Retrieved from the Internet <URL:https://web.archive.org/web/20170803120554/https://missiontherapeutics.com/pipeline/usp30-inhibitor-programs/>
- N. NAKAMURA ET AL: "Regulation of Mitochondrial Morphology by USP30, a Deubiquitinating Enzyme Present in the Mitochondrial Outer Membrane", MOLECULAR BIOLOGY OF THE CELL, vol. 19, no. 5, 1 May 2008 (2008-05-01), pages 1903 - 1911, XP055092951, ISSN: 1059-1524, DOI: 10.1091/mbc.E07-11-1103
- CAI YU ET AL: "Smurf2, an E3 ubiquitin ligase, interacts with PDE4B and attenuates liver fibrosis through miR-132 mediated CTGF inhibition", BIOCHIMICA ET BIOPHYSICA ACTA. MOLECULAR CELL RESEARCH, vol. 1865, no. 2, 31 October 2017 (2017-10-31), pages 297 - 308, XP085326462, ISSN: 0167-4889, DOI: 10.1016/J.BBAMCR.2017.10.011

## Description

The present invention relates to novel treatments of fibrotic diseases, including, *inter alia,* fibrosis of the lung, liver and kidney, and to substituted-cyanopyrrolidines having activity as inhibitors of ubiquitin specific peptidase 30 (USP30), and compositions containing said inhibitors, for use in said treatments.

Ubiquitin is a small protein consisting of 76 amino acids that is important for the regulation of protein function in the cell. Ubiquitylation and deubiquitylation are enzymatically mediated processes by which ubiquitin is covalently bound or cleaved from a target protein by deubiquitylating enzymes (DUBs), of which there are approximately 95 DUBs in human cells, divided into sub-families based on sequence homology. The USP family are characterised by their common Cys and His boxes which contain Cys and His residues critical for their DUB activities. The ubiquitylation and deubiquitylation processes have been implicated in the regulation of many cellular functions including cell cycle progression, apoptosis, modification of cell surface receptors, regulation of DNA transcription and DNA repair. Thus, the ubiquitin system has been implicated in the pathogenesis of numerous disease states including inflammation, viral infection, metabolic dysfunction, CNS disorders, and oncogenesis.

Ubiquitin is a master regulator of mitochondrial dynamics. Mitochondria are dynamic organelles whose biogenesis, fusion and fission events are regulated by the post-translational regulation via ubiquitylation of many key factors such as mitofusins. While ubiquitin ligases such as parkin are known to ubiquitylate a number of mitochondrial proteins, until recently, deubiquitylating enzymes remained elusive. USP30 is a 517 amino acid protein which is found in the mitochondrial outer membrane (Nakamura et al., Mol Biol 19:1903-11, 2008). It is the sole deubiquitylating enzyme bearing a mitochondrial addressing signal and has been shown to deubiquitylate a number of mitochondrial proteins. It has been demonstrated that USP30 opposes parkin-mediated mitophagy and that reduction of USP30 activity can rescue parkin-mediated defects in mitophagy (Bingol, B. et al., "", Nature Vol. 510, 370-375, 19 Jun 2014.

Mitochondrial dysfunction can be defined as diminished mitochondrial content (mitophagy or mitochondrial biogenesis), as a decrease in mitochondrial activity and oxidative phosphorylation, but also as modulation of reactive oxygen species (ROS) generation. Hence a role for mitochondrial dysfunctions in a very large number of aging processes and pathologies including but not limited to, neurodegenerative diseases (e.g. Parkinson's disease (PD), Alzheimer's disease, Huntington's disease, Amylotrophic Lateral Sclerosis (ALS), multiple sclerosis), cancer, diabetes, metabolic disorders, cardio-vascular diseases, psychiatric diseases (e.g. Schizophrenia), and osteoarthritis.

For example, Parkinson's disease affects around 10 million people worldwide (Parkinson's Disease Foundation) and is characterised by the loss of dopaminergic neurons in the substantia nigra. The exact mechanisms underlying PD are unclear; however mitochondrial dysfunction is increasingly appreciated as a key determinant of dopaminergic neuronal susceptibility in PD and is a feature of both familial and sporadic disease, as well as in toxin-induced Parkinsonism. Parkin is one of a number of proteins that have been implicated with early onset PD. While most PD cases are linked to defects in alpha-synuclein, 10% of Parkinson's cases are linked to specific genetic defects, one of which is in the ubiquitin E3 ligase parkin. Parkin and the protein kinase PTEN-induced putative kinase 1 (PINK1) collaborate to ubiquitylate mitochondrial membrane proteins of damaged mitochondria resulting in mitophagy. Dysregulation of mitophagy results in increased oxidative stress, which has been described as a characteristic of PD. Inhibition of USP30 could therefore be a potential strategy for the treatment of PD. For example, PD patients with parkin mutations leading to reduced activity could be therapeutically compensated by inhibition of USP30.

It has been reported that depletion of USP30 enhances mitophagic clearance of mitochondria and also enhances parkin-induced cell death. USP30 has also been shown to regulate BAX/BAK-dependent apoptosis independently of parkin over expression. Depletion of USP30 sensitises cancer cells to BH-3 mimetics such as ABT-737, without the need for parkin over expression. Thus, an anti-apoptotic role has been demonstrated for USP30 and USP30 is therefore a potential target for anti-cancer therapy.

The ubiquitin-proteasome system has gained interest as a target for the treatment of cancer following the approval of the proteasome inhibitor bortezomib (Velcade^{®}) for the treatment of multiple myeloma. Extended treatment with bortezomib is limited by its associated toxicity and drug resistance. However, therapeutic strategies that target specific aspects of the ubiquitin-proteasome pathway upstream of the proteaseome, such as DUBs, are predicted to be better tolerated (Bedford et al., Nature Rev 10:29-46, 2011).

Fibrotic diseases, including renal, hepatic and pulmonary fibrosis, are a leading cause of morbidity and mortality and can affect all tissues and organ systems. Fibrosis is considered to be the result of acute or chronic stress on the tissue or organ, characterized by extracellular matrix deposition, reduction of vascular/tubule/duct/airway patency and impairment of function ultimately resulting in organ failure. Many fibrotic conditions are promoted by lifestyle or environmental factors; however, a proportion of fibrotic conditions can be initiated through genetic triggers or indeed are considered idiopathic (i.e. without a known cause). Certain fibrotic disease, such as idiopathic pulmonary fibrosis (IPF), can be treated with non-specific kinase inhibitor (nintedanib) or drugs without a well-characterized mechanism of action (pirfenidone). Other treatments for organ fibrosis, such as kidney or liver fibrosis, alleviate pressure on the organ itself (e.g. beta blockers for cirrhosis, angiotensin receptor blockers for chronic kidney disease). Attention to lifestyle factors, such as glucose and diet control, may also influence the course and severity of disease.

Preclinical models are available to study potential novel therapeutics, through their ability to model fibrosis pathology (e.g. collagen deposition) consistent with the human condition. Preclinical models can be toxin-mediated (e.g. bleomycin for lung and skin fibrosis), surgical (e.g. unilateral ureter obstruction model for acute tubulointerstitial fibrosis), and genetic (e.g. diabetic (db/db) mice for diabetic nephropathy). For example, both examples previously given for indicated IPF treatments (nintedanib and pirfenidone) show efficacy in the bleomycin lung fibrosis model.

Mitochondrial dysfunction has been implicated in a number of fibrotic diseases, with oxidative stress downstream of dysfunction being the key pathogenic mediator, alongside decreased ATP production. In preclinical models, disruption of the mitophagy pathway (through mutation or knockout of either parkin or PINK1) exacerbates lung fibrosis and kidney fibrosis, with evidence of increased oxidative stress.

Kurita Y., et al., "Pirfenidone inhibits myofibroblast differentiation and lung fibrosis development during insufficient mitophagy", Respiratory Research (2017) 18:114, discloses that accumulation of profibrotic myofibroblasts is a crucial process for fibrotic remodelling in IPF. Recent findings are said to show participation of autophagy/mitophagy, part of the lysosomal degradation machinery, in IPF pathogenesis, and that mitophagy has been implicated in myofibroblast differentiation through regulating mitochondrial reactive oxygen species (ROS)-mediated platelet-derived growth factor receptor (PDGFR) activation. Kurita's results suggested that pirfenidone induces PARK2-mediated mitophagy and also inhibits lung fibrosis development in the setting of insufficient mitophagy, which may at least partly explain the anti-fibrotic mechanisms for IPF treatment.

Williams J.A. et.al. "Targeting Pink1-Parkin-Mediated Mitophagy for Treating Liver Injury", Pharmacol Res. 2015 December; 102: 264-269, discuss the role of Pink1-Parkin-mediated autophagy in protecting against alcohol and acetaminophen-induced liver injury by removing damaged mitochondria via mitophagy. It is suggested that pharmacological stabilization of USP8 or inactivation of USP15 and USP30 may be potential therapeutic targets for upregulating Parkin-induced mitophagy and in turn protect against drug-induced liver injury. However, it is noted that the DUBs are regulated both transcriptionally and post-translationally, which may make drug development for targeting these specific enzymes challenging, and in addition, phosphorylated ubiquitin was shown to be resistant to DUBs. The authors conclude that upregulating PINK1 stabilization or kinase activity may be a more effective target than inhibiting DUBs.

Williams J.A. et.al. "A Mechanistic Review of Mitophagy and Its Role in Protection against Alcoholic Liver Disease", Biomolecules 2015, 5, 2619-2642, and "Parkin regulates mitophagy and mitochondrial function to protect against alcohol-induced liver injury and steatosis in mice", Am J Physiol Gastrointest Liver Physiol 309: G324-G340, 2015, reviews mechanisms involved in regulation of mitochondrial homeostasis in the liver and how these mechanisms may protect against alcohol-induced liver disease.

Series of derivatives of cyano-substituted heterocycles are disclosed as deubiquitylating enzyme inhibitors in PCT applications WO 2016/046530, WO 2016/156816, WO 2017/009650, WO 2017/093718, WO 2017/103614, WO 2017/149313, WO 2017/109488, WO 2017/141036, WO 2017/163078, WO 2017/158381, WO 2017/158388, PCT/GB2017/052971, PCT/GB2017/052949, PCT/GB2017/052880, and PCT/GB2017/052882. Falgueyret et al., J.Med.Chem. 2001, 44, 94-104, and PCT application WO 01/77073 refer to cyanopyrrolidines as inhibitors of Cathepsins K and L, with potential utility in treating osteoporosis and other bone-resorption related conditions. PCT application WO 2015/179190 refers to N-acylethanolamine hydrolysing acid amidase inhibitors, with potential utility in treating ulcerative colitis and Crohn's disease. PCT application WO 2013/030218 refers to quinazolin-4-one compounds as inhibitors of ubiquitin specific proteases, such as USP7, with potential utility in treating cancer, neurodegenerative diseases, inflammatory disorders and viral infections. PCT applications WO 2015/017502 and WO 2016/019237 refer to inhibitors of Bruton's tyrosine kinase with potential utility in treating disease such as autoimmune disease, inflammatory disease and cancer. PCT applications WO 2009/026197, WO 2009/129365, WO 2009/129370, and WO 2009/129371, refer to cyanopyrrolidines as inhibitors of Cathepsin C with potential utility in treating COPD. United States patent application US 2008/0300268 refers to polyaromatic compounds as inhibitors of tyrosine kinase receptor PDGFR. Lonergan D., PCT application WO 2015/183987, refers to pharmaceutical compositions comprising deubiquitinase inhibitors and human serum albumin in methods of treating cancer, fibrosis, an autoimmune disease or condition, an inflammatory disease or condition, a neurodegenerative disease or condition or an infection. Lonergan notes that deubiquitinases, including UCHL5/UCH37, USP4, USP9X, USP11 and USP15, are said to have been implicated in the regulation of the TGF-beta signalling pathway, the disruption of which gives rise to neurodegenerative and fibrotic diseases, autoimmune dysfunction and cancer.

PCT application WO 2006/067165 refers to a method for treating fibrotic diseases using indolinone kinase inhibitors. PCT application WO 2007/119214 refers to a method for treating early stage pulmonary fibrosis using an endothelin receptor antagonist. PCT application WO 2012/170290 refers to a method for treating fibrotic diseases using THC acids. PCT application WO 2018/213150 refers sulphonamide USP30 inhibitors with potential utility in the treatment of conditions involving mitochondrial defects. Larson-Casey et al., 2016, Immunity 44, 582-596, concerns macrophage Akt1 kinase-mediated mitophagy, apoptosis resistance and pulmonary fibrosis. Tang et al., Kidney Diseases 2015, 1, 71-79, reviews the potential role of mitophagy in renal pathophysiology.

Mora Ana L. ET AL ("Mitochondria in the spotlight of aging and idiopathic pulmonary fibrosis", The Journal of Clinical Investigation, vol. 127, no. 2, 1 February 2017 (2017-02-01), pages 405-414) teaches that mitochondrial dysfunction is involved in fibrotic diseases and that therapeutic approaches targeting that may be beneficial for halting the progression of disease and improving quality of life in IPF patients.

There exists a need for a safe, alternative and/or improved methods and compositions for the treatment of fibrotic disorders and the various symptoms and conditions associated therewith. Unexpectedly, it has now been found that the substituted-cyanopyrrolidines of the present invention may be suitable for treating fibrotic diseases. While not wishing to be bound by any particular theory or mechanism, it is believed that the compounds of the present invention act to inhibit the enzyme USP30, which in turn upregulates Parkin-induced mitophagy.

According to a first aspect, the present invention provides a compound of formula (I): 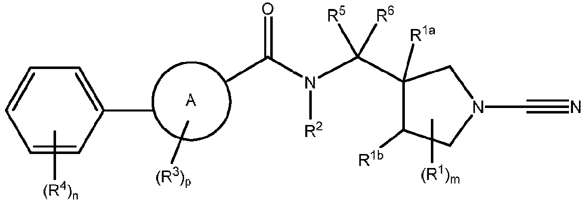 a tautomer thereof, or a pharmaceutically acceptable salt of said compound or tautomer, for use in the treatment of fibrosis, wherein:
m is 0 to 5;
n is 0 to 5;
p is 0 to 3;
ring A is a monocyclic heteroaryl ring containing 1, 2 or 3 heteroatoms, each independently selected from N, O and S;
each R¹ is independently selected from halo, cyano, hydroxy, (C₁-C₆)alkyl, (C₁-C₆)alkoxy, halo(C₁-C₆)alkyl, halo(C₁-C₆)alkoxy, and (C₁-C₆)alkoxy(C₁-C₆)alkyl;
R^{1a} and R^{1b}, are each independently selected from hydrogen, halo, cyano, hydroxy, (C₁-C₆)alkyl, (C₁-C₆)alkoxy, halo(C₁-C₆)alkyl, halo(C₁-C₆)alkoxy, and (C₁-C₆)alkoxy(C₁-C₆)alkyl;
R² is selected from hydrogen, (C₁-C₆)alkyl, and (C₁-C₆)alkoxy(C₁-C₆)alkyl;
each R³ is independently selected from hydrogen, halo, cyano, hydroxy, (C₁-C₆)alkyl, (C₁-C₆)alkoxy, (C₁-C₆)alkoxy(C₁-C₆)alkyl, halo(C₁-C₆)alkyl, halo(C₁-C₆)alkoxy, NH(C₁-C₆)alkyl, N((C₁-C₆)alkyl)₂, C(O)NH(C₁-C₆)alkyl, C(O)N((C₁-C₆)alkyl)₂, NHC(O)(C₁-C₆)alkyl, N((C₁-C₆)alkyl)C(O)(C₁-C₆)alkyl), C(O)(C₁-C₆)alkyl, C(O)O(C₁-C₆)alkyl, CO₂H, CONH₂, SO₂NH(C₁-C₆)alkyl, and SO₂N((C₁-C₆)alkyl)₂;
each R⁴ is independently selected from halo, cyano, hydroxy, (C₁-C₆)alkyl, (C₁-C₆)alkoxy, (C₁-C₆)alkoxy(C₁-C₆)alkyl, halo(C₁-C₆)alkyl, halo(C₁-C₆)alkoxy, NH(C₁-C₆)alkyl, N((C₁-C₆)alkyl)₂, C(O)NH(C₁-C₆)alkyl, C(O)N((C₁-C₆)alkyl)₂, NHC(O)(C₁-C₆)alkyl, N((C₁-C₆)alkyl)C(O)(C₁-C₆)alkyl), C(O)(C₁-C₆)alkyl, C(O)O(C₁-C₆)alkyl, CO₂H, CONH₂, SO₂NH(C₁-C₆)alkyl, and SO₂N((C₁-C₆)alkyl)₂; and
R⁵ and R⁶ are each independently selected from hydrogen, cyano, and (C₁-C₆)alkyl; or R⁵ and R⁶ together form a 3 to 6 membered cycloalkyl ring.

Unless otherwise indicated, alkyl, and alkoxy groups, including the corresponding divalent radicals, may be straight or branched and contain 1 to 6 carbon atoms and typically 1 to 4 carbon atoms. Examples of alkyl include methyl, ethyl, n-propyl, isopropyl, n-butyl, isobutyl, sec-butyl, pentyl and hexyl. Examples of alkoxy include methoxy, ethoxy, isopropoxy and n-butoxy. Cycloalkyl includes, for example, cyclopropyl.

Halo means fluoro, chloro, bromo or iodo, in particular, fluoro or chloro. Haloalkyl and haloalkoxy groups may contain one or more halo substituents. Examples are trifluoromethyl and trifluoromethoxy.

Heteroaryl means a polyunsaturated, monocyclic 5-membered aromatic moiety containing at least one and up to 3 heteroatoms, particularly 1, 2 or 3 heteroatoms selected from N, O and S, and the remaining ring atoms are carbon atoms. Heteroaryl ring nitrogen and sulfur atoms are optionally oxidised, and the nitrogen atom(s) are optionally quaternized. The point of attachment of heteroaryl to the group it is a substituent of can be via a carbon atom or a heteroatom (e.g. nitrogen). Examples of ring A include pyrrolyl, furanyl, thiophenyl, pyrazolyl, imidazolyl, isoxazolyl, oxazolyl, isothiazolyl, thiazolyl, triazolyl (including 1,2,3-triazolyl and 1,2,4-triazolyl), oxadiazolyl (including 1-oxa-2,3-diazolyl, 1-oxa-2,4-diazolyl, 1-oxa-2,5-diazolyl, and 1-oxa-3,4-diazolyl), and thiadiazolyl (including 1-thia-2,4-diazolyl, 1-thia-2,5-diazolyl, and 1-thia-3,4-diazolyl).

Unless otherwise indicated, the term substituted means substituted by one or more defined groups. In the case where groups may be selected from more than one alternatives, the selected groups may be the same or different. The term independently means that where more than one substituent is selected from more than one possible substituents, those substituents may be the same or different.

Preferred embodiments of the compound of formula (I) for use in the present invention are defined below.

Preferably, m is selected from 0, 1, 2, 3 and 4. More preferably, m is 0, 1 or 2. Most preferably, m is 0 or 1;
Preferably, n is selected from 0, 1, 2, 3, and 4. Most preferably, n is 0, 1 or 2.

Preferably, p is selected from 0, 1, and 2. Most preferably, p is 0 or 1.

Preferably, ring A is selected from pyrrolyl, furanyl, thiophenyl, pyrazolyl, imidazolyl, isoxazolyl, oxazolyl, isothiazolyl, thiazolyl, triazolyl, oxadiazolyl, and thiadiazolyl.

More preferably, ring A is selected from pyrazolyl, imidazolyl, isoxazolyl, oxazolyl, isothiazolyl, thiazolyl, triazolyl, oxadiazolyl, and thiadiazolyl.

Most preferably, ring A is selected from pyrazolyl, imidazolyl, isoxazolyl, oxazolyl, thiazolyl, triazolyl, and oxadiazolyl.

Preferably, each R¹ is independently selected from halo, cyano, hydroxy, (C₁-C₃)alkyl, (C₁-C₃)alkoxy, halo(C₁-C₃)alkyl, halo(C₁-C₃)alkoxy, and (C₁-C₃)alkoxy(C₁-C₃)alkyl. More preferably, each R¹ is independently selected from chloro, fluoro, methyl, ethyl, and methoxy. Most preferably, R¹ is methyl.

Preferably, R^{1a} is selected from hydrogen, halo, cyano, hydroxy, (C₁-C₃)alkyl, (C₁-C₃)alkoxy, halo(C₁-C₃)alkyl, halo(C₁-C₃)alkoxy, and (C₁-C₃)alkoxy(C₁-C₃)alkyl. More preferably, R^{1a} is selected from hydrogen, chloro, fluoro, cyano, hydroxy, methyl, ethyl, methoxy, and methoxymethyl. Yet more preferably, R^{1a} is selected from hydrogen and fluoro. Most preferably, R^{1a} is hydrogen.

Preferably, R^{1b} is selected from hydrogen, halo, cyano, hydroxy, (C₁-C₃)alkyl, (C₁-C₃)alkoxy, halo(C₁-C₃)alkyl, halo(C₁-C₃)alkoxy, and (C₁-C₃)alkoxy(C₁-C₃)alkyl. More preferably, R^{1b} is selected from hydrogen, chloro, fluoro, cyano, hydroxy, methyl, ethyl, methoxy, and methoxymethyl. Yet more preferably, R^{1b} is selected from hydrogen, hydroxy and methyl. Most preferably, R^{1b} is selected from hydrogen and methyl.

In one preferred embodiment, m is 0, R^{1a} is hydrogen, and R^{1b} is methyl.

In another preferred embodiment, m is 1, R¹ is methyl, and R^{1a} and R^{1b} are each hydrogen.

In another preferred embodiment, m is 0, and R^{1a} and R^{1b} are each hydrogen.

Preferably, R² is selected from hydrogen, (C₁-C₃)alkyl, and (C₁-C₃)alkoxy(C₁-C₃)alkyl. More preferably, R² is selected from hydrogen, methyl, ethyl, propyl, and methoxyethyl. Yet more preferably, R² is selected from hydrogen, methyl, and ethyl. Most preferably, R² is selected from hydrogen and methyl.

Preferably, each R³ is independently selected from hydrogen, halo, cyano, hydroxy, (C₁-C₃)alkyl, (C₁-C₃)alkoxy, (C₁-C₃)alkoxy(C₁-C₃)alkyl, halo(C₁-C₃)alkyl, halo(C₁-C₃)alkoxy, NH(C₁-C₃)alkyl, N((C₁-C₃)alkyl)₂, C(O)NH(C₁-C₃)alkyl, C(O)N((C₁-C₃)alkyl)₂, NHC(O)(C₁-C₃)alkyl, N((C₁-C₃)alkyl)C(O)(C₁-C₃)alkyl), C(O)(C₁-C₃)alkyl, C(O)O(C₁-C₃)alkyl, CO₂H, CONH₂, SO₂NH(C₁-C₃)alkyl, and SO₂N((C₁-C₃)alkyl)₂.

More preferably, each R³ is independently selected from hydrogen, chloro, fluoro, cyano, hydroxy, (C₁-C₃)alkyl, (C₁-C₃)alkoxy, (C₁-C₃)alkoxy(C₁-C₃)alkyl, halo(C₁-C₃)alkyl, and halo(C₁-C₃)alkoxy. Yet more preferably, each R³ is independently selected from hydrogen, chloro, fluoro, cyano, hydroxy, methyl, ethyl, propyl, methoxy, ethoxy, and methoxymethyl. Most preferably, each R³ is independently selected from hydrogen and methyl.

Preferably, each R⁴ is independently selected from halo, cyano, hydroxy, (C₁-C₃)alkyl, (C₁-C₃)alkoxy, (C₁-C₃)alkoxy(C₁-C₃)alkyl, halo(C₁-C₃)alkyl, halo(C₁-C₃)alkoxy, NH(C₁-C₃)alkyl, N((C₁-C₃)alkyl)₂, C(O)NH(C₁-C₃)alkyl, C(O)N((C₁-C₃)alkyl)₂, NHC(O)(C₁-C₃)alkyl, N((C₁-C₃)alkyl)C(O)(C₁-C₃)alkyl), C(O)(C₁-C₃)alkyl, C(O)O(C₁-C₃)alkyl, CO₂H, CONH₂, SO₂NH(C₁-C₃)alkyl, and SO₂N((C₁-C₃)alkyl)₂.

More preferably, each R⁴ is independently selected from chloro, fluoro, cyano, hydroxy, (C₁-C₃)alkyl, (C₁-C₃)alkoxy, (C₁-C₃)alkoxy(C₁-C₃)alkyl, halo(C₁-C₃)alkyl, and halo(C₁-C₃)alkoxy. Yet more preferably, each R⁴ is independently selected from chloro, fluoro, cyano, hydroxy, methyl, ethyl, propyl, methoxy, ethoxy, methoxymethyl, CF₃, and OCF₃. Most preferably, each R⁴ is independently selected from chloro, fluoro, cyano, methyl, methoxy, and CF₃.

Preferably, R⁵ and R⁶ are each independently selected from hydrogen, cyano, methyl, ethyl, and propyl; or R⁵ and R⁶ together form a cyclopropyl ring. More preferably, R⁵ and R⁶ are each independently selected from hydrogen, cyano, and methyl. More preferably, R⁵ is selected from hydrogen and methyl. Most preferably, R⁵ is hydrogen. More preferably, R⁶ is selected from hydrogen, cyano and methyl. Most preferably, R⁶ is hydrogen. Most preferably, R⁵ and R⁶ are each hydrogen.

According to a first preferred aspect of the invention:
m is 0, 1 or 2;
n is 0, 1 or 2;
p is 0 or 1;
ring A is a monocyclic heteroaryl ring containing 1, 2 or 3 heteroatoms, each independently selected from N, O and S;
each R¹ is independently selected from halo, cyano, hydroxy, (C₁-C₃)alkyl, (C₁-C₃)alkoxy, halo(C₁-C₃)alkyl, halo(C₁-C₃)alkoxy, and (C₁-C₃)alkoxy(C₁-C₃)alkyl;
R^{1a} is selected from hydrogen, halo, cyano, hydroxy, (C₁-C₃)alkyl, (C₁-C₃)alkoxy, halo(C₁-C₃)alkyl, halo(C₁-C₃)alkoxy, and (C₁-C₃)alkoxy(C₁-C₃)alkyl;
R^{1b} is selected from hydrogen, halo, cyano, hydroxy, (C₁-C₃)alkyl, (C₁-C₃)alkoxy, halo(C₁-C₃)alkyl, halo(C₁-C₃)alkoxy, and (C₁-C₃)alkoxy(C₁-C₃)alkyl;
R² is selected from hydrogen, (C₁-C₃)alkyl, and (C₁-C₃)alkoxy(C₁-C₃)alkyl;
each R³ is independently selected from hydrogen, chloro, fluoro, cyano, hydroxy, (C₁-C₃)alkyl, (C₁-C₃)alkoxy, (C₁-C₃)alkoxy(C₁-C₃)alkyl, halo(C₁-C₃)alkyl, and halo(C₁-C₃)alkoxy; and
each R⁴ is independently selected from chloro, fluoro, cyano, hydroxy, (C₁-C₃)alkyl, (C₁-C₃)alkoxy, (C₁-C₃)alkoxy(C₁-C₃)alkyl, halo(C₁-C₃)alkyl, and halo(C₁-C₃)alkoxy; and
R⁵ and R⁶ are each independently selected from hydrogen, cyano, methyl, ethyl, and propyl; or R⁵ and R⁶ together form a cyclopropyl ring;
a tautomer thereof, or a pharmaceutically acceptable salt of said compound or tautomer.

According to a more preferred aspect of the invention:
m is 0 or 1;
n is 1 or 2;
p is 0 or 1;
ring A is a monocyclic heteroaryl ring containing 1, 2 or 3 heteroatoms, each independently selected from N, O and S;
R¹ is selected from chloro, fluoro, methyl, ethyl, and methoxy;
R^{1a} is selected from hydrogen, chloro, fluoro, cyano, hydroxy, methyl, ethyl, methoxy, and methoxymethyl;
R^{1b} is selected from hydrogen, chloro, fluoro, cyano, hydroxy, methyl, ethyl, methoxy, and methoxymethyl;
R² is selected from hydrogen, methyl, ethyl, propyl, and methoxyethyl;
each R³ is independently selected from hydrogen, chloro, fluoro, cyano, hydroxy, methyl, ethyl, propyl, methoxy, ethoxy, and methoxymethyl; and
each R⁴ is independently selected from chloro, fluoro, cyano, hydroxy, methyl, ethyl, propyl, methoxy, ethoxy, methoxymethyl, CF₃, and OCF₃; and
R⁵ and R⁶ are each independently selected from hydrogen, cyano, methyl, ethyl, and propyl; or R⁵ and R⁶ together form a cyclopropyl ring;
a tautomer thereof, or a pharmaceutically acceptable salt of said compound or tautomer.

According to a yet more preferred aspect of the invention:
m is 0 or 1;
n is 1 or 2;
p is 0 or 1;
ring A is a monocyclic heteroaryl ring containing 1, 2 or 3 heteroatoms, each independently selected from N, O and S;
R¹ is methyl;
R^{1a} is selected from hydrogen and fluoro;
R^{1b} is selected from hydrogen and methyl;
R² is selected from hydrogen and methyl;
each R³ is independently selected from hydrogen and methyl; and
each R⁴ is independently selected from chloro and cyano;
R⁵ is selected from hydrogen and methyl; and
R⁶ is selected from hydrogen, cyano and methyl;
a tautomer thereof, or a pharmaceutically acceptable salt of said compound or tautomer.

According to a yet more preferred aspect of the invention:
m is 0;
n is 1 or 2;
p is 0 or 1;
ring A is selected from pyrazolyl, imidazolyl, isoxazolyl, oxazolyl, thiazolyl, triazolyl, and oxadiazolyl;
R^{1a} is hydrogen;
R^{1b} is selected from hydrogen and methyl;
R² is selected from hydrogen and methyl;
R³ is selected from hydrogen and methyl; and
each R⁴ is independently selected from chloro and cyano; and
R⁵ and R⁶ are each hydrogen;
a tautomer thereof, or a pharmaceutically acceptable salt of said compound or tautomer.

According to one example of the first aspect of the invention, the compound of formula (I) is a compound of formula (Ia): 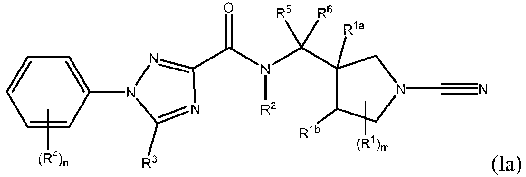 wherein m, n, R¹, R^{1a}, R^{1b}, R², R³, R⁴, R⁵, and R⁶, are as defined in respect of the first aspect of the invention and preferred embodiments thereof; a tautomer thereof, or a pharmaceutically acceptable salt of said compound or tautomer.

According to another example of the first aspect of the invention, the compound of formula (I) is a compound of formula (Ib): 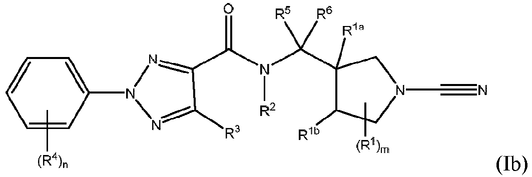 wherein m, n, R¹, R^{1a}, R^{1b}, R², R³, R⁴, R⁵, and R⁶, are as defined in respect of the first aspect of the invention and preferred embodiments thereof; a tautomer thereof, or a pharmaceutically acceptable salt of said compound or tautomer.

According to another example of the first aspect of the invention, the compound of formula (I) is a compound of formula (Ic): 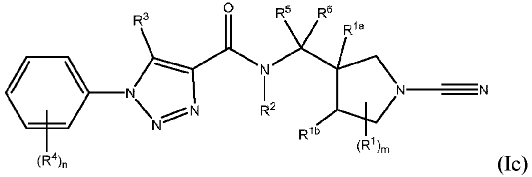 wherein m, n, R¹, R^{1a}, R^{1b}, R², R³, R⁴, R⁵, and R⁶, are as defined in respect of the first aspect of the invention and preferred embodiments thereof; a tautomer thereof, or a pharmaceutically acceptable salt of said compound or tautomer.

According to another example of the first aspect of the invention, the compound of formula (I) is a compound of formula (Id): 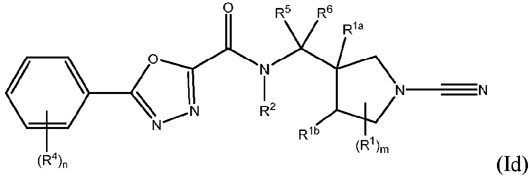 wherein m, n, R¹, R^{1a}, R^{1b}, R², R⁴, R⁵, and R⁶, are as defined in respect of the first aspect of the invention and preferred embodiments thereof; a tautomer thereof, or a pharmaceutically acceptable salt of said compound or tautomer.

According to another example of the first aspect of the invention, the compound of formula (I) is a compound of formula (Ie): 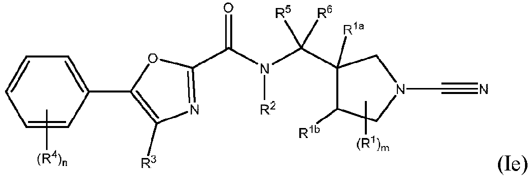 wherein m, n, R¹, R^{1a}, R^{1b}, R², R³, R⁴, R⁵, and R⁶, are as defined in respect of the first aspect of the invention and preferred embodiments thereof, a tautomer thereof, or a pharmaceutically acceptable salt of said compound or tautomer.

According to another example of the first aspect of the invention, the compound of formula (I) is a compound of formula (If): 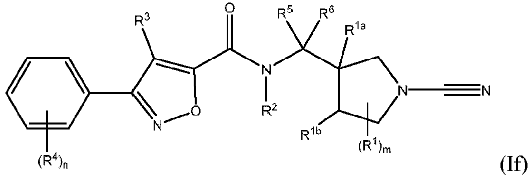 wherein m, n, R¹, R^{1a}, R^{1b}, R², R³, R⁴, R⁵, and R⁶, are as defined in respect of the first aspect of the invention and preferred embodiments thereof; a tautomer thereof, or a pharmaceutically acceptable salt of said compound or tautomer.

According to another example of the first aspect of the invention, the compound of formula (I) is a compound of formula (Ig): 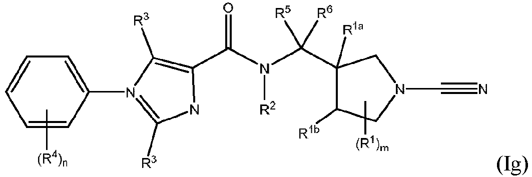 wherein m, n, R¹, R^{1a}, R^{1b}, R², R³, R⁴, R⁵, and R⁶, are as defined in respect of the first aspect of the invention and preferred embodiments thereof; a tautomer thereof, or a pharmaceutically acceptable salt of said compound or tautomer.

In one particularly preferred embodiment of each of the compounds of formula (I), (Ia), (Ib), (Ic), (Id), (Ie), (If), and (Ig):
m is 0, R^{1a} is hydrogen, R^{1b} is methyl, and n, R², R³, R⁴, R⁵, and R⁶, are as defined in respect of the first aspect of the invention and preferred embodiments thereof.

In another particularly preferred embodiment of each of the compounds of formula (I), (Ia), (Ib), (Ic), (Id), (Ie), (If), and (Ig):
m is 1, R¹ is methyl, R^{1a} and R^{1b} are each hydrogen, and n, R², R³, R⁴, R⁵, and R⁶, are as defined in respect of the first aspect of the invention and preferred embodiments thereof.

In another particularly preferred embodiment of each of the compounds of formula (I), (Ia), (Ib), (Ic), (Id), (Ie), (If), and (Ig):
m is 0, R^{1a} and R^{1b} are each hydrogen, and n, R², R³, R⁴, R⁵, and R⁶, are as defined in respect of the first aspect of the invention and preferred embodiments thereof.

Preferred compounds of formula (I) for use in the present invention are selected from:
1-(3-cyanophenyl)-N-((1-cyanopyrrolidin-3-yl)methyl)-1H-1,2,4-triazole-3-carboxamide;
(R)-1-(3-cyanophenyl)-N-((1-cyanopyrrolidin-3-yl)methyl)-1H-1,2,4-triazole-3-carboxamide (Example 1);
(S)-1-(3-cyanophenyl)-N-((1-cyanopyrrolidin-3-yl)methyl)-1H-1,2,4-triazole-3-carboxamide;
1-(3-cyanophenyl)-N-((1-cyanopyrrolidin-3-yl)methyl)-N-methyl-1H-1,2,4-triazole-3-carboxamide;
(R)-1-(3-cyanophenyl)-N-((1-cyanopyrrolidin-3-yl)methyl)-N-methyl-1H-1,2,4-triazole-3-carboxamide;
(S)-1-(3-cyanophenyl)-N-((1-cyanopyrrolidin-3-yl)methyl)-N-methyl-1H-1,2,4-triazole-3-carboxamide (Example 2);
N-((1-cyanopyrrolidin-3-yl)methyl)-1-phenyl-1H-1,2,4-triazole-3-carboxamide (Example 3);
(R)-N-((1-cyanopyrrolidin-3-yl)methyl)-1-phenyl-1H-1,2,4-triazole-3-carboxamide;
(S)-N-((1-cyanopyrrolidin-3-yl)methyl)-1-phenyl-1H-1,2,4-triazole-3-carboxamide;
N-(((2S,3S)-1-cyano-2-methylpyrrolidin-3-yl)methyl)-1-(3-cyanophenyl)-1H-1,2,4-triazole-3-carboxamide;
N-(((2S,3R)-1-cyano-2-methylpyrrolidin-3-yl)methyl)-1-(3-cyanophenyl)-1H-1,2,4-triazole-3-carboxamide;
N-(((2R)-1-cyano-2-methylpyrrolidin-3-yl)methyl)-1-(3-cyanophenyl)-1H-1,2,4-triazole-3-carboxamide (Example 4);
N-(((2R,3R)-1-cyano-2-methylpyrrolidin-3-yl)methyl)-1-(3-cyanophenyl)-1H-1,2,4-triazole-3-carboxamide (diastereoisomer of Example 4);
N-(((2R,3S)-1-cyano-2-methylpyrrolidin-3-yl)methyl)-1-(3-cyanophenyl)-1H-1,2,4-triazole-3-carboxamide (diastereoisomer of Example 4);
N-(((2S)-1-cyano-2-methylpyrrolidin-3-yl)methyl)-1-(3-cyanophenyl)-1H-1,2,4-triazole-3-carboxamide;
1-(3-cyanophenyl)-N-((1-cyanopyrrolidin-3-yl)methyl)-1H-1,2,3-triazole-4-carboxamide;
(R)-1-(3-cyanophenyl)-N-((1-cyanopyrrolidin-3-yl)methyl)-1H-1,2,3-triazole-4-carboxamide (Example 5);
(S)-1-(3-cyanophenyl)-N-((1-cyanopyrrolidin-3-yl)methyl)-1H-1,2,3-triazole-4-carboxamide;
1-(3-chlorophenyl)-N-((1-cyanopyrrolidin-3-yl)methyl)-1H-1,2,3-triazole-4-carboxamide;
(R)-1-(3-chlorophenyl)-N-((1-cyanopyrrolidin-3-yl)methyl)-1H-1,2,3-triazole-4-carboxamide (Example 6);
(S)-1-(3-chlorophenyl)-N-((1-cyanopyrrolidin-3-yl)methyl)-1H-1,2,3-triazole-4-carboxamide;
2-(3-chlorophenyl)-N-((1-cyanopyrrolidin-3-yl)methyl)-2H-1,2,3-triazole-4-carboxamide;
(R)-2-(3-chlorophenyl)-N-((1-cyanopyrrolidin-3-yl)methyl)-2H-1,2,3-triazole-4-carboxamide (Example 7);
(S)-2-(3-chlorophenyl)-N-((1-cyanopyrrolidin-3-yl)methyl)-2H-1,2,3-triazole-4-carboxamide;

Preferred compounds of formula (I) for use in the present invention are selected from the following examples as described in WO 2017/103614:
(R)-3-(((5-phenylthiazol-2-yl)amino)methyl)pyrrolidine-1-carbonitrile (Ex. 9);
(S)-3-(((5-phenylthiazol-2-yl)amino)methyl)pyrrolidine-1-carbonitrile (Ex. 10);
N-((1-cyanopyrrolidin-3-yl)methyl)-2-phenyloxazole-5-carboxamide (Ex. 20);
N-((1-cyanopyrrolidin-3-yl)methyl)-3-phenylisoxazole-5-carboxamide (Ex. 21);
N-((1-cyanopyrrolidin-3-yl)methyl)-5-phenyl-1H-pyrazole-3-carboxamide (Ex. 22);
N-((1-cyanopyrrolidin-3-yl)methyl)-3-(o-tolyl)-1H-pyrazole-5-carboxamide (Ex. 25);
N-((1-cyanopyrrolidin-3-yl)methyl)-2-phenylthiazole-4-carboxamide (Ex. 26);
N-((1-cyanopyrrolidin-3-yl)methyl)-3-(2-fluorophenyl)-1H-pyrazole-5-carboxamide (Ex. 31);
N-((1-cyanopyrrolidin-3-yl)methyl)-5-phenyloxazole-2-carboxamide (Ex. 38);
(R)-3-(3-chlorophenyl)-N-((1-cyanopyrrolidin-3-yl)methyl)isoxazole-5-carboxamide (Ex. 55);
(R)-N-((1-cyanopyrrolidin-3-yl)methyl)-5-phenylisoxazole-3-carboxamide (Ex. 58);
(R)-N-((1-cyanopyrrolidin-3-yl)methyl)-5-phenylthiazole-2-carboxamide (Ex. 61);
(R)-N-((1-cyanopyrrolidin-3-yl)methyl)-4-phenylthiazole-2-carboxamide (Ex. 62);
(R)-N-((1-cyanopyrrolidin-3-yl)methyl)-1-phenyl-1H-pyrazole-3-carboxamide (Ex. 63);
(R)-N-((1-cyanopyrrolidin-3-yl)methyl)-2-phenyl-1H-imidazole-5-carboxamide (Ex. 64);
(R)-3-(2-chlorophenyl)-N-((1-cyanopyrrolidin-3-yl)methyl)isoxazole-5-carboxamide (Ex. 66);
(R)-3-(4-chlorophenyl)-N-((1-cyanopyrrolidin-3-yl)methyl)isoxazole-5-carboxamide (Ex. 67);
(R)-5-(3-cyanophenyl)-N-((1-cyanopyrrolidin-3-yl)methyl)-1,3,4-oxadiazole-2-carboxamide (Ex. 68);
(S)-5-(3-cyanophenyl)-N-((1-cyanopyrrolidin-3-yl)methyl)-1,3,4-oxadiazole-2-carboxamide (Ex. 69);
(R)-N-((1-cyanopyrrolidin-3-yl)methyl)-1-phenyl-1H-imidazole-4-carboxamide (Ex. 70);
(R)-1-(3-cyanophenyl)-N-((1-cyanopyrrolidin-3-yl)methyl)-1H-imidazole-4-carboxamide (Ex. 71);
(R)-1-(4-cyanophenyl)-N-((1-cyanopyrrolidin-3-yl)methyl)-1H-imidazole-4-carboxamide (Ex. 72);
(R)-N-((1-cyanopyrrolidin-3-yl)methyl)-1-(2-methoxyphenyl)-1H-imidazole-4-carboxamide (Ex. 73); and
(R)-N-((1-cyanopyrrolidin-3-yl)methyl)-1-(3-methoxyphenyl)-1H-imidazole-4-carboxamide (Ex. 74);
a tautomer thereof, or a pharmaceutically acceptable salt of said compound or tautomer.

Pharmaceutical acceptable salts of the compounds of formula (I) include the acid addition and base salts (including di-salts) thereof.

Suitable acid addition salts are formed from acids which form non-toxic salts.

Examples include the acetate, aspartate, benzoate, besylate, bicarbonate/carbonate, bisulfate, camsylate, citrate, edisylate, esylate, fumarate, gluceptate, gluconate, glucuronate, hibenzate, hydrochloride/chloride, hydrobromide/bromide, hydroiodide/iodide, hydrogen phosphate, isethionate, D- and L-lactate, malate, maleate, malonate, mesylate, methylsulfate, 2-napsylate, nicotinate, nitrate, orotate, palmate, phosphate, saccharate, stearate, succinate sulfate, D-and L-tartrate, and tosylate salts.

A pharmaceutical acceptable salt of a compound of formula (I) may be readily prepared by mixing together solutions of the compound of formula (I) and the desired acid or base, as appropriate. The salt may precipitate from solution and be collected by filtration or may be recovered by evaporation of the solvent.

Pharmaceutical acceptable solvates in accordance with the invention include hydrates and solvates wherein the solvent of crystallization may be isotopically substituted, e. g. D₂O, acetone-d₆, DMSO-d₆. Also within the scope of the invention are clathrates, drug-host inclusion complexes wherein, in contrast to the aforementioned solvates, the drug and host are present in non-stoichiometric amounts. For a review of such complexes, see J.Pharm Sci, 64 (8), 1269-1288 by Haleblian (August 1975).

Hereinafter all references to compounds of formula (I) include references to salts thereof and to solvates and clathrates of compounds of formula (I) and salts thereof.

The invention includes all polymorphs of the compounds of formula (I) as hereinbefore defined.

Certain derivatives of compounds of formula (I) which have little or no pharmacological activity themselves can, when metabolised upon administration into or onto the body, give rise to compounds of formula (I) having the desired activity. Such derivatives are referred to as "prodrugs".

Prodrugs can, for example, be produced by replacing appropriate functionalities present in the compounds of formula (I) with certain moieties known to those skilled in the art as "pro-moieties" as described, for example, in "Design of Prodrugs" by H Bundgaard (Elsevier, 1985).

Finally, certain compounds of formula (I) may themselves act as prodrugs of other compounds of formula (I).

Certain derivatives of compounds of formula (I) which contain a nitrogen atom may also form the corresponding N-oxide.

Compounds of formula (I) containing one or more asymmetric carbon atoms can exist as two or more optical isomers. Where a compound of formula (I) contains an alkenyl or alkenylene group, geometric *cis*/*trans* (or *Z*/*E*) isomers are possible, and where the compound contains, for example, a keto or oxime group, tautomeric isomerism ('tautomerism') may occur. It follows that a single compound may exhibit more than one type of isomerism.

Included within the scope of the present invention are all optical isomers, geometric isomers and tautomeric forms of the compounds of formula, including compounds exhibiting more than one type of isomerism, and mixtures of one or more thereof.

*Cis*/*trans* isomers may be separated by conventional techniques well known to those skilled in the art, for example, fractional crystallisation and chromatography.

Conventional techniques for the preparation/isolation of individual enantiomers include chiral synthesis from a suitable optically pure precursor or resolution of the racemate (or the racemate of a salt or derivative) using, for example, chiral high performance liquid chromatography (HPLC). Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture. The present invention includes all crystal forms of the compounds of formula (I) including racemates and racemic mixtures (conglomerates) thereof. Stereoisomeric conglomerates may be separated by conventional techniques known to those skilled in the art - see, for example, "Stereochemistry of Organic Compounds" by E. L. Eliel and S. H. Wilen (Wiley, New York, 1994).

In particular, the compounds of formula (I) contain a chiral centre at the carbon atom of the pyrrolidine ring that is substituted by R^{1a}, and said stereocentre can thus exist in either the (*R*) or (*S*) configuration. The designation of the absolute configuration (*R*) and (*S*) for stereoisomers in accordance with IUPAC nomenclature is dependent on the nature of the substituents and application of the sequence-rule procedure. The compounds of formula (I) may thus exist in either of the following enantiomeric configurations:

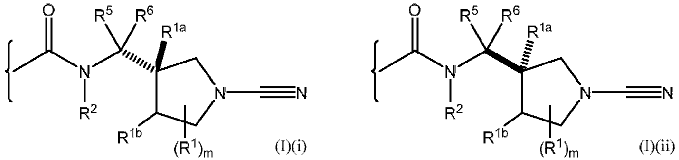

In a preferred aspect, the compounds of formula (I) possess the absolute stereochemical configuration:

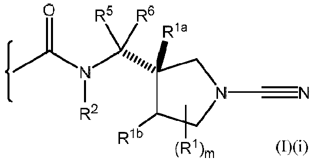

In another preferred aspect the compounds of formula (I) possess the absolute stereochemical configuration:

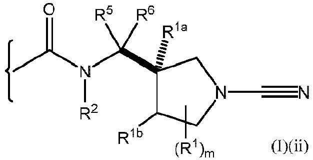

Included within the scope of the present invention are each of these (*R*) and (*S*) stereoisomers of the compounds of formula (I) in individual form; formula (I)(i), Formula (I)(ii), or mixtures thereof. When the compound of formula (I) is isolated as a single stereoisomer, the compound may exist with an enantiomeric excess of at least 80%, preferably at least 90%, more preferably at least 95%, for example 96%, 97%, 98%, 99%, or 100%.

The present invention also includes all pharmaceutically acceptable isotopic variations of a compound of formula (I). An isotopic variation is defined as one in which at least one atom is replaced by an atom having the same atomic number, but an atomic mass different from the atomic mass usually found in nature.

Examples of isotopes suitable for inclusion in the compounds of the invention include isotopes of hydrogen, such as ²H and ³H, carbon, such as ¹³C and ¹⁴C, nitrogen, such as ¹⁵N, oxygen, such as ¹⁷O and ¹⁸O, phosphorus, such as ³²P, sulphur, such as ³⁵S, fluorine, such as ¹⁸F, and chlorine, such as ³⁶CI.

Substitution of the compounds of the invention with isotopes such as deuterium may afford certain therapeutic advantages resulting from greater metabolic stability, for example, increased in vivo half-life or reduced dosage requirements, and hence may be preferred in some circumstances.

Certain isotopic variations of the compounds of formula (I), for example, those incorporating a radioactive isotope, are useful in drug and/or substrate tissue distribution studies. The radioactive isotopes tritium, and ¹⁴C, are particularly useful for this purpose in view of their ease of incorporation and ready means of detection.

Isotopic variations of the compounds of formula (l) can generally be prepared by conventional techniques known to those skilled in the art or by processes analogous to those described in the accompanying Examples and Preparations using appropriate isotopic variations of suitable reagents.

According to a further aspect, the present invention provides a compound of formula (I) as defined herein, a tautomer thereof, or a pharmaceutically acceptable salt of said compound or tautomer for use in the treatment of fibrosis, wherein said compound is administered orally.

According to a further aspect, the present invention provides a pharmaceutically acceptable composition comprising a compound of formula (I) as defined herein, a tautomer thereof, or a pharmaceutically acceptable salt of said compound or tautomer, for use in the treatment of fibrosis.

According to a further aspect, the present invention provides a pharmaceutically acceptable composition comprising a compound of formula (I) as defined herein, a tautomer thereof, or a pharmaceutically acceptable salt of said compound or tautomer, for use in the treatment of fibrosis, wherein said compound is administered orally, intravenously, sublingually, or buccally.

According to a further aspect, the present invention provides a pharmaceutically acceptable composition comprising a compound of formula (I) as defined herein, a tautomer thereof, or a pharmaceutically acceptable salt of said compound or tautomer, wherein said compound is administered orally.

According to a further aspect, the present invention provides the use of a compound of formula (I) as defined herein, a tautomer thereof, or a pharmaceutically acceptable salt of said compound or tautomer, in the preparation of a medicament for the treatment of fibrosis.

According to a further aspect, the present invention provides the use of a compound of formula (I) as defined herein, a tautomer thereof, or a pharmaceutically acceptable salt of said compound or tautomer, in the preparation of a medicament for the treatment of fibrosis, wherein the medicament is adapted for oral, intravenous, sublingual, or buccal administration.

According to a further aspect, the present invention provides the use of a compound of formula (I) as defined herein, a tautomer thereof, or a pharmaceutically acceptable salt of said compound or tautomer, in the preparation of a medicament for the treatment of fibrosis, wherein the medicament is adapted for oral administration.

Fibrosis refers to the accumulation of extracellular matrix constituents that occurs following trauma, inflammation, tissue repair, immunological reactions, cellular hyperplasia, and neoplasia. Fibrotic disorders that may be treated by the compounds and compositions of the present invention include, *inter alia,* fibrosis/fibrotic disorders associated with major organ diseases, for example, interstitial lung disease (ILD), liver cirrhosis, non-alcoholic fatty liver disease (NAFLD) and non-alcoholic steatohepatitis (NASH) (hepatic fibrosis), kidney disease (renal fibrosis), heart or vascular disease (cardiac fibrosis) and diseases of the eye; fibroproliferative disorders, for example, systemic and local scleroderma, keloids and hypertrophic scars, atherosclerosis, restenosis, and Dupuytren's contracture; scarring associated with trauma, for example, surgical complications, chemotherapeutics drug-induced fibrosis (e.g. bleomycin-induced fibrosis), radiation-induced fibrosis, accidental injury and bums); retroperitoneal fibrosis (Ormond's disease); and peritoneal fibrosis/peritoneal scarring in patients receiving peritoneal dialysis, usually following renal transplantation. See, for example, Wynn, Thomas A., "Fibrotic disease and the TH1/TH2 paradigm", Nat Rev Immunol. 2004 August ; 4(8): 583-594. The present invention therefore relates to methods of treatment, and compounds and compositions used in said methods of fibrosis/fibrotic disorders of and/or associated with the major organs, the lung, liver, kidney, heart, skin, eye, gastrointestinal tract, peritoneum, bone marrow, etc., and other diseases/disorders herein described.

Interstitial lung disease (ILD) includes disorders in which pulmonary inflammation and fibrosis are the final common pathways of pathology, for example, sarcoidosis, silicosis, drug reactions, infections and collagen vascular diseases, such as rheumatoid arthritis and systemic sclerosis (scleroderma). The fibrotic disorder of the lung includes, for example, pulmonary fibrosis, idiopathic pulmonary fibrosis (IPF), usual interstitial pneumonitis (UIP), interstitial lung disease, cryptogenic fibrosing alveolitis (CFA), bronchiolitis obliterans, and bronchiectasis.

Idiopathic pulmonary fibrosis (IPF) is the most common type of ILD and has no known cause.

Liver cirrhosis has similar causes to ILD and includes, for example, cirrhosis associated with viral hepatitis, schistosomiasis and chronic alcoholism.

Kidney disease, may be associated with diabetes, which can damage and scar the kidneys leading to a progressive loss of function, and also hypertensive diseases. Kidney fibrosis may occur at any stage of kidney disease, from chronic kidney disease (CKD) through to end-stage renal disease (ESRD). Kidney fibrosis can develop as a result of cardiovascular disease such as hypertension or diabetes, both of which place immense strain on kidney function which promotes a fibrotic response. However, kidney fibrosis can also be idiopathic (without a known cause), and certain genetic mitochondrial diseases also present kidney fibrosis manifestations and associated symptoms.

Heart disease may result in scar tissue that can impair the ability of the heart to pump.

Diseases of the eye include, for example, macular degeneration and retinal and vitreal retinopathy, which can impair vision.

In a preferred embodiment, the present invention is directed to the treatment of Idiopathic pulmonary fibrosis (IPF).

In another preferred embodiment, the present invention is directed to the treatment of kidney fibrosis.

References to 'treatment' includes curative, palliative and prophylactic, and includes means to ameliorate, alleviate symptoms, eliminate the causation of the symptoms either on a temporary or permanent basis, or to prevent or slow the appearance of symptoms of the named disorder or condition. It will be understood that certain conditions are not treated prophylactically and therefore treatment occurs upon diagnosis of a disorder or condition. The compounds of the invention are useful in the treatment of humans and other mammals.

Pharmaceutical compositions of the invention comprise any of the compounds of the invention combined with any pharmaceutically acceptable carrier, adjuvant or vehicle. Examples of pharmaceutically acceptable carriers are known to those skilled in the art and include, but are not limited to, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may be in the form of, for example, tablets, capsules, powders, granules, elixirs, lozenges, suppositories, syrups and liquid preparations including suspensions and solutions. The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms.

The compounds of the invention or pharmaceutical compositions thereof, as described herein, may be used alone or combined with one or more additional pharmaceutical agents. For example, the compounds of the invention or pharmaceutical compositions thereof, as described herein, may be used alone or combined with one or more additional pharmaceutical agents selected from the group consisting of anticholinergic agents, beta-2 mimetics, steroids, PDE-IV inhibitors, p38 MAP kinase inhibitors, NKi antagonists, LTD4 antagonists, EGFR inhibitors and endothelin antagonists.

In particular, the compounds of the invention or pharmaceutical compositions thereof, as described herein, may be used alone or combined with one or more additional pharmaceutical agents selected from the group consisting of general immunosuppressive drugs, such as a corticosteroid, immunosuppressive or cytotoxic agents, or antifibrotics, such as pirfenidone or a non-specific kinase inhibitor (e.g. nintedanib).

The pharmaceutical compositions of the invention may be administered in any suitably effective manner, such as oral, parenteral, topical, inhaled, intranasal, rectal, intravaginal, ocular, and andial. Pharmaceutical compositions suitable for the delivery of compounds of the present invention and methods for their preparation will be readily apparent to those skilled in the art. Such compositions and methods for their preparation may be found, for example, in "Remington's Pharmaceutical Sciences", 19th Edition (Mack Publishing Company, 1995).

### Oral Administration

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi-and nano-particulates, gels, films (including muco- adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast- disintegrating dosage forms such as those described in Expert Opinion in Therapeutic Patents, 11 (6), 981-986 by Liang and Chen (2001).

A typical tablet may be prepared using standard processes known to a formulation chemist, for example, by direct compression, granulation (dry, wet, or melt), melt congealing, or extrusion. The tablet formulation may comprise one or more layers and may be coated or uncoated.

Examples of excipients suitable for oral administration include carriers, for example, cellulose, calcium carbonate, dibasic calcium phosphate, mannitol and sodium citrate, granulation binders, for example, polyvinylpyrrolidine, hydroxypropylcellulose, hydroxypropylmethylcellulose and gelatin, disintegrants, for example, sodium starch glycolat and silicates, lubricating agents, for example, magnesium stearate and stearic acid, wetting agents, for example, sodium lauryl sulphate, preservatives, anti-oxidants, flavours and colourants.

Solid formulations for oral administration may be formulated to be immediate and/or modified release. Modified release formulations include delayed-, sustained-, pulsed-, controlled dual-, targeted and programmed release. Details of suitable modified release technologies such as high energy dispersions, osmotic and coated particles are to be found in Verma et al, Pharmaceutical Technology On-line, 25 (2), 1-14 (2001). Other modified release formulations are described in US Patent No. 6,106, 864.

### Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by suitable processing, for example, the use of high energy spray-dried dispersions (see WO 01/47495) and/or by the use of appropriate formulation techniques, such as the use of solubility-enhancing agents.

Formulations for parenteral administration may be formulated to be immediate and/or modified release. Modified release formulations include delayed-, sustained-, pulsed-, controlled dual-, targeted and programmed release.

Pharmaceutical compositions of the present invention also include compositions and methods known in the art for bypassing the blood brain barrier or can be injected directly into the brain. Suitable areas for injection include the cerebral cortex, cerebellum, midbrain, brainstem, hypothalamus, spinal cord and ventricular tissue, and areas of the PNS including the carotid body and the adrenal medulla.

### Dosage

The magnitude of an effective dose of a compound will, of course, vary with the nature of the severity of the condition to be treated and the route of administration. The selection of appropriate dosages is within the remit of the physician. The daily dose range is about 10µg to about 100 mg per kg body weight of a human and non-human animal and in general may be around 10µg to 30mg per kg body weight per dose. The above dose may be given from one to three times per day.

For example, oral administration may require a total daily dose of from 5mg to 1000mg, such as from 5 to 500mg, while an intravenous dose may only require from 0.01 to 30 mg/kg body weight, such as from 0.1 to 10 mg/kg, more preferably from 0.1 to 1 mg/kg body weight. The total daily dose may be administered in single or divided doses.

The skilled person will also appreciate that, in the treatment of certain conditions, compounds of the invention may be taken as a single dose on an "as required" basis (i.e. as needed or desired).

### Synthetic methodologies

Compounds of formula (I) may be prepared as described in WO 2017/103614 using the general methods and experimental of the representative examples. Additionally, compounds of formula (I), in particular compounds of formula (Ia), (Ib), and (Ic), may be prepared using methods as described below in the general reaction schemes and the representative examples. Where appropriate, the individual transformations within a scheme may be completed in a different order.

According to a further aspect, the present invention provides a process for the preparation of a compound of formula (I), as defined herein [(Ia), (Ib), (Ic)], comprising reacting a compound of formula (IVa), (IVb), or (IVc), as appropriate, where X is OH with an amine of formula (V), where PG is a protecting group, such as BOC or CBZ, to give an amide of formula (IIIa), (IIIb), or (IIIc) (Scheme 1). The amide-coupling reaction can be performed using standard methodology, for example by reaction using a coupling reagent such as DCC, HATU, HBTU, EDC or via a mixed anhydride. Alternatively, the acids (IVa), (IVb), or (IVc), where X is OH, can be converted into the acid chlorides (IVa), (IVb), or (IVc), where X is Cl, using SOCl₂, PCl₃, or PCl₅, which can then be reacted with the amine (V), preferably in a suitable solvent in the presence of a suitable base. Alternatively, the compound (IVa), (IVb), or (IVc), where X forms the ester, can be reacted directly with the amine (V), preferably in a suitable solvent.

Additionally, one compound of formula (IIIa), (IIIb), or (IIIc) may be converted into another compound of formula (IIIa), (IIIb), or (IIIc), for example *via* a Suzuki coupling of a bromo-aryl or bromo-heteroaryl group. The compound of formula (IIIa), (IIIb), or (IIIc), may be deprotected using standard methods to give amine (IIa), (IIb), or (IIc), which may then be reacted with cyanogen bromide to give the corresponding compound of formula (I). Scheme 1 illustrates one method that may be used, as applied to the compound of formula (Ia) using the compound of formula (IVa), which applies equally to the preparation of the compounds of formula (Ib) and formula (Ic) using the appropriate corresponding compounds of formula (IVb) and (IVc).

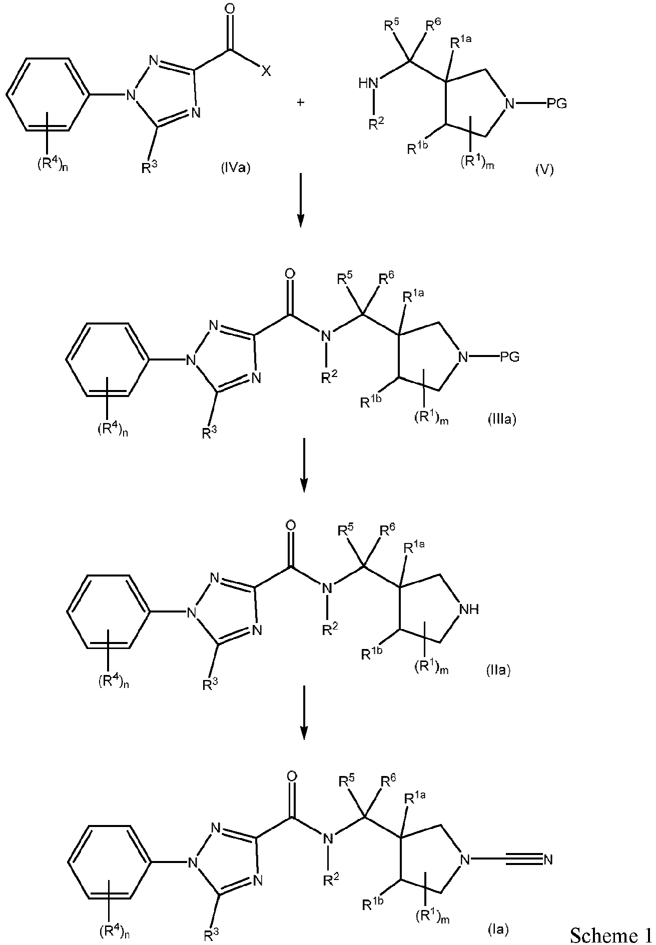

Additional representative compounds and stereoisomers, racemic mixtures, diastereomers and enantiomers thereof may be prepared using the intermediates prepared in accordance to the general schemes and other materials, compounds and reagents known to those skilled in the art. Enantiomers may be separated using standard techniques, such as Chiral HPLC, for example, using column CHIRALART SA 250x4.6mm 5 µm.

The invention is illustrated by the following non-limiting examples in which the following abbreviations and definitions are used.

### Abbreviations

- ACN: Acetonitrile
- d: Doublet (NMR signal)
- DBU: 1,8-Diazabicyclo[5.4.0]undec-7-ene
- DCM: Dichloromethane
- DIPEA: N,N-Diisopropylamine
- DMF: N,N-Dimethylformamide
- DMS: Dimethyl sulphide
- DMSO: Dimethylsulphoxide
- EDC: 1-Ethyl-3-(3-dimethylaminopropyl)carbodiimide
- ES: Electrospray
- EtOAc: Ethyl acetate
- h: Hour(s)
- HATU: 1-[Bis(dimethylamino)methylene]-1H-1,2,3-triazolo[4,5-b]pyridinium 3-oxid hexafluorophosphate
- LCMS: Liquid Chromatography-Mass Spectrometry
- m: Multiplet (NMR signal)
- MeOH: Methanol
- min: Minute(s)
- NMR: Nuclear Magnetic Resonance
- rt: Room temperature
- s: Singlet (NMR signal)
- SFC: Supercritical fluid chromatography
- TBD: 1,5,7-Triazabicyclo[4.4.0]dec-5-ene
- TFA: Trifluoroacetic acid
- THF: Tetrahydrofuran

### Analytical Methods

### LCMS Methods:

| **Method B** | | |
|---|---|---|
| Column | X-bridge C18, 50×4.6mm, 3.5µm or equivalent | |
| Mobile Phase | 0.1% Ammonia in Water | |
| | 0.1% Ammonia in Acetonitrile | |
| Flow Rate | 1.0 mL/min | |
| Gradient | Time | %B |
| | 0.01 | 5 |
| | 5.00 | 90 |
| | 5.80 | 95 |
| | 7.20 | 95 |
| | 7.21 | 5 |
| | 10.00 | 5 |

| **Method C** | | |
|---|---|---|
| Column | BEH C18, 50×2.1mm, 1.7µm or equivalent | |
| Mobile Phase | 5mM Ammonium Acetate + 0.1% Formic Acid in Water 0.1% Formic Acid in Acetonitrile | |
| Flow Rate | 0.55 mL/min | |
| Gradient | Time | %B |
| | 0.01 | 5 |
| | 0.40 | 5 |
| | 0.80 | 35 |
| | 1.20 | 55 |
| | 2.50 | 100 |
| | 3.30 | 100 |
| | 3.31 | 5 |
| | 4.00 | 5 |

| **Method H** | | |
|---|---|---|
| Column | X-bridge C18, 50x4.6mm, 3.5µm or equivalent | |
| Mobile Phase | (A) 0.1% Ammonia in Water | |
| | (B) 0.1% Ammonia in Acetonitrile | |
| Flow Rate | 1.0 mL/min | |
| Gradient | Time | %B |
| | 0.01 | 5 |
| | 5.00 | 90 |
| | 5.80 | 95 |
| | 7.20 | 95 |
| | 7.21 | 5 |
| | 10.00 | 5 |

| **Method J** | | |
|---|---|---|
| Column | BEH C18, 50*2.1mm, 1.7µm or equivalent | |
| Mobile Phase | (A) SmM Ammonium Acetate + 0.1% Formic Acid in Water | |
| | (B) 0.1% Formic Acid in Acetonitrile | |
| Flow Rate | 0.45 mL/min | |
| Gradient | Time | %B |
| | 0.01 | 2 |
| | 0.50 | 2 |
| | 5.00 | 90 |
| | 6.00 | 95 |
| | 7.00 | 95 |
| | 7.01 | 2 |
| | 8.00 | 2 |

| **Method M** | | |
|---|---|---|
| Column | BEH C18, 50*2.1mm, 1.7µm or equivalent | |
| Mobile Phase | (A) SmM Ammonium Acetate + 0.1% Formic Acid in Water | |
| | (B) 0.1% Formic Acid in Acetonitrile | |
| Flow Rate | 0.40 mL/min | |
| Gradient | Time | %B |
| | 0.01 | 10 |
| | 5.00 | 90 |
| | 7.00 | 100 |
| | 11.00 | 100 |
| | 11.01 | 10 |
| | 12.00 | 10 |

### Long run LCMS Methods:

| **Method D-FA** | | |
|---|---|---|
| Mobile phase | A | 0.1%formic acid in water |
| | B | 0.1% formic acid in acetonitrile |
| Instrument | | Agilent 1290 Infinity RRLC attached with Agilent 6120 Mass detector and Diode array Detector |
| Column | | SUNFIRE C18,5µ,4.6 * 250 mm,Waters |
| Flow rate | | 1.0 mL/min |
| Column oven temperature | | Ambient |
| Run time | | 30.0 min |
| Flow rate | 1.0 mL/min | |
| Gradient: | Time | %B |
| | 0.0 | 0 |
| | 15.0 | 20 |
| | 25.0 | 100 |
| | 27.0 | 100 |
| | 27.01 | 0 |
| | 30.0 | 0 |

| **Method D-NH₃** | | |
|---|---|---|
| Mobile phase | A | 0.1% Ammonia in water |
| | B | 0.1% Ammonia in Acetonitrile |
| Instrument | | Agilent 1290 Infinity RRLC attached with Agilent 6120 Mass detector and Diode array Detector |
| Column | | Waters, X Bridge, C18,250 * 4.6mm |
| Flow rate | | 1.0 mL/min |
| Column oven temperature | | Ambient |
| Run time | | 40.0 min |
| Gradient: | | |
| | Time | %B |
| | 0.00 | 5 |
| | 5.00 | 5 |
| | 10.00 | 30 |
| | 15.00 | 30 |
| | 25.00 | 60 |
| | 30.00 | 90 |
| | 35.00 | 90 |
| | 35.01 | 5 |
| | 40.00 | 0 |

| **Method D-AA** | | |
|---|---|---|
| Mobile phase | A | 10mM ammonium acetate in water |
| | B | 100 % Acetonitrile |
| Instrument | | Agilent 1290 Infinity RRLC attached with Agilent 6120 Mass detector and Diode array Detector |
| Column | | YMC EXRS 250 C18,4.6 * 250mm |
| Flow rate | | 1.0 mL/min |
| Column oven temperature | | Ambient |
| Run time | | 30.0 min |
| Gradient: | Time | %B |
| | 0.0 | 5 |
| | 15.0 | 50 |
| | 20.0 | 50 |
| | 25.0 | 100 |
| | 27.0 | 100 |
| | 27.01 | 5 |
| | 30.0 | 5 |

### Example 1

### (R)-1-(3-cyanophenyl)-N-((1-cyanopyrrolidin-3-yl)methyl)-1H-1,2,4-triazole-3-carboxamide

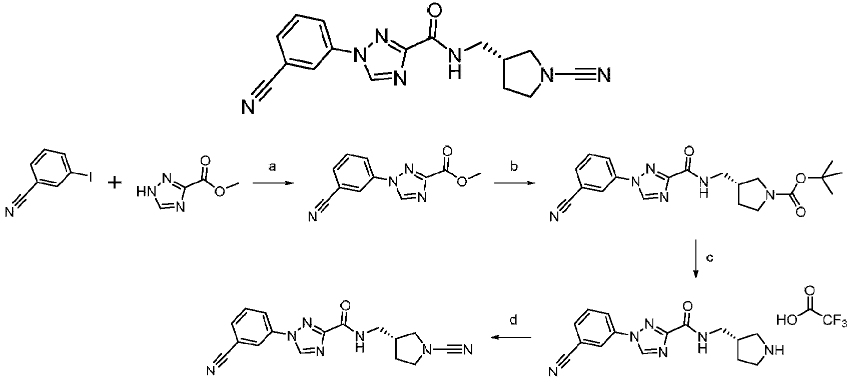

**Step a.** To a stirred solution of 3-iodobenzonitrile (0.500 g, 2.18 mmol) and methyl 1H-1,2,4-triazole-3-carboxylate (CAS Number 4928-88-5; 0.277 g, 2.18 mmol) in DMSO (6 mL) was added L-proline (0.050 g, 0.44 mmol), Cu(I)I (0.083 g, 0.44 mmol) and K₂CO₃ (0.602 g, 4.34 mmol) at rt. The solution was heated at 80°C for 18 h. The reaction mixture was cooled to rt and poured into ice cold water (100 mL) and extracted with EtOAc (4 x 50 mL). Combined organic extracts were dried over anhydrous sodium sulfate, filtered and concentrated under reduced pressure. The residue was purified by flash column chromatography (compound eluted in 35% EtOAc in hexane) yielding methyl 1-(3-cyanophenyl)-1H-1,2,4-triazole-3-carboxylate (0.105 g, 0.46 mmol). LCMS: Method H, 1.517 min, MS: ES+ 229.19.

**Step b.** To a stirred solution of methyl 1-(3-cyanophenyl)-1H-1,2,4-triazole-3-carboxylate (0.100 g, 0.44 mmol) and tert-butyl (R)-3-(aminomethyl)pyrrolidine-1-carboxylate (CAS Number 199174-29-3; 0.106 g, 0.53 mmol) in THF (4 mL) was added a solution of 1,5,7-triazabicyclo[4.4.0]dec-5-ene (CAS Number 5807-14-7; 0.092 g, 0.66 mmol) in THF (1 mL) at 0°C. The resulting mixture was stirred at rt for 18h. The reaction mixture was poured into water (50 mL) and extracted with EtOAc (2 x 50 mL). Combined organic layers were dried over anhydrous sodium sulfate, filtered and concentrated under reduced pressure. The residue was purified by flash column chromatography (compound eluted in 5% MeOH in DCM) yielding tert-butyl (R)-3-((1-(3-cyanophenyl)-1H-1,2,4-triazole-3-carboxamido)methyl)-pyrrolidine-1-carboxylate. (0.083 g, 0.21 mmol), LCMS: Method H, 1.789 min, MS: ES+ [M-100] 297.26.

**Step c.** To a stirred solution of tert-butyl (R)-3-((1-(3-cyanophenyl)-1H-1,2,4-triazole-3-carboxamido)-methyl)pyrrolidine-1-carboxylate (0.075 g, 0.189 mmol) in DCM (4 mL) was added TFA (0.37 mL) at 0°C and stirred for 1.5 h. Volatiles were distilled off under reduced pressure and the residue azeotropically distilled with DCM (3 x 50 mL) and dried under high vacuum yielding (S)-1-(3-cyanophenyl)-N-(pyrrolidin-3-ylmethyl)-1H-1,2,4-triazole-3-carboxamide TFA salt, 0.110 g, crude. LCMS: Method H, 1.331 min, MS: ES+ 297.26.

**Step d.** To a stirred solution of (S)-1-(3-cyanophenyl)-N-(pyrrolidin-3-ylmethyl)-1H-1,2,4-triazole-3-carboxamide TFA salt (0.100 g, crude from previous step) in THF (5 mL) was added K₂CO₃ (0.100 g, 0.73 mmol) at 0°C. After stirring at 0°C for 15 min, cyanogen bromide (0.026 g, 0.24 mmol) was added and stirring continued for a further 1 h. The resulting mixture was poured into water (50 mL) and extracted with EtOAc (2 x 50 mL). Combined organic extracts were dried over anhydrous sodium sulfate, filtered and concentrated under reduced pressure. The residue was purified by flash column chromatography (compound eluted in 95% EtOAc in hexane) yielding (R)-1-(3-cyanophenyl)-N-((1-cyanopyrrolidin-3-yl)methyl)-1H-1,2,4-triazole-3-carboxamide (0.030 g, 0.09 mmol). LCMS: Method J, 2.767 min, MS: ES- 320.2; ¹H NMR (400 MHz, DMSO-d6) δ ppm: 9.49 (s, 1 H), 8.93 - 8.96 (t, J= 6.0 Hz, 1 H), 8.44 (s, 1 H), 8.23 - 8.25 (m, 1 H), 7.93 - 7.95 (d, J= 7.6 Hz 1 H), 7.78 - 7.82 (m, 1 H), 3.34 - 3.42 (m, 3 H), 3.14 - 3.29 (m, 4 H), 1.89 - 1.94 (m, 1 H), 1.63 - 1.68 (m, 1 H).

### Example 2

### (S)-1-(3-cyanophenyl)-N-((1-cyanopyrrolidin-3-yl)methyl)-N-methyl-1H-1,2,4-triazole-3-carboxamide

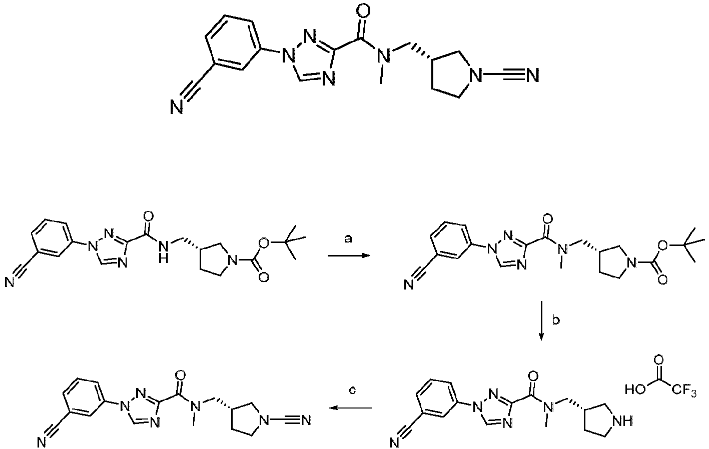

**Step a.** To a stirred solution of tert-butyl (R)-3-((1-(3-cyanophenyl)-1H-1,2,4-triazole-3-carboxamido)-methyl)pyrrolidine-1-carboxylate (described in **Example 1,** steps a and b; 0.100 g, 0.25 mmol) in DMF (2 mL) was added NaH (60% in mineral oil; 0.008 g, 0.30 mmol) portion-wise at 0°C. After stirring for 15 min, methyl iodide (0.035 g, 0.25 mmol) was added and the resulting reaction mixture was stirred at rt for 1h. The reaction was poured into ice water (50 mL) and extracted with EtOAc (2 x 50 mL). The combined organic phase was dried over anhydrous sodium sulfate, filtered and concentrated under reduced pressure. The resulting residue was purified by flash column chromatography (eluted in 2% MeOH in DCM) yielding tert-butyl (R)-3-((1-(3-cyanophenyl)-N-methyl-1H-1,2,4-triazole-3-carboxamido)methyl)-pyrrolidine-1-carboxylate (0.095 g, 0.23 mmol). LCMS: Method H, 1.755 min, MS: ES+ 411.4.

**Step b.** A stirred solution of tert-butyl (R)-3-((1-(3-cyanophenyl)-N-methyl-1H-1,2,4-triazole-3-carboxamido)methyl)pyrrolidine-1-carboxylate (0.090 g, 0.22 mmol) in DCM (5 mL) was added TFA (0.45 mL) at 0°C. The reaction mixture was stirred at 0°C to rt for 1.5h. The resulting reaction mixture was concentrated under reduced pressure. The obtained residue was azeotropically distilled with DCM (3 x 50 mL) and dried under high vacuum yielding (S)-1-(3-cyanophenyl)-N-methyl-N-(pyrrolidin-3-ylmethyl)-1H-1,2,4-triazole-3-carboxamide TFA salt, 0.110 g, crude. LCMS: Method H, 1.325 min, MS: ES+ 311.28.

**Step c.** To a stirred solution of (S)-1-(3-cyanophenyl)-N-methyl-N-(pyrrolidin-3-ylmethyl)-1H-1,2,4-triazole-3-carboxamide TFA salt (0.100 g, crude from previous step) in THF (5 mL) was added K₂CO₃ (0.097 g, 0.71 mmol) at 0°C. After stirring for 10 min, cyanogen bromide (0.024 g, 0.24 mmol) was added and stirring continued for 1h at rt. The resulting mixture was poured into water (50 mL) and extracted with EtOAc (2 x 50 mL). The combined organic phase was dried over anhydrous sodium sulfate, filtered and concentrated under reduced pressure. The residue was purified by flash column chromatography (compound eluted in 2% MeOH in DCM) yielding (S)-1-(3-cyanophenyl)-N-((1-cyanopyrrolidin-3-yl)methyl)-N-methyl-1H-1,2,4-triazole-3-carboxamide (0.028 g, 0.08 mmol). LCMS: Method J, 2.723 min, MS: ES+ 336.15; ¹H NMR (400 MHz, DMSO-d6) δ ppm: 9.48 (s, 1 H), 8.42 - 8.43 (d, J= 4.0 Hz, 1H), 8.19 - 823 (m, 1H), 7.92 - 7.94 (d, J= 8.0 Hz, 1 H), 7.77 - 7.81 (t, J= 8 Hz 1 H), 3.44 - 3.58 (m, 3 H), 3.36 - 3.41 (m, 1 H), 3.23 - 3.27 (m, 1 H), 2.99 - 3.10 (m, 4 H), 2.59 - 2.65 (m, 1 H), 1.86 - 1.89 (m, 1 H), 1.64 - 1.69 (m, 1 H).

### Example 3

### N-((1-cyanopyrrolidin-3-yl)methyl)-1-phenyl-1H-1,2,4-triazole-3-carboxamide

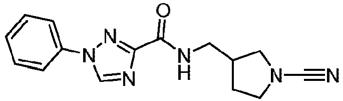

Prepared using analogous procedures to those described herein.

### Example 4

### N-(((2R, 3R)-1-cyano-2-methylpyrrolidin-3-yl)methyl)-1-(3-cyanophenyl)-1H-1,2, 4-triazole-3-carboxamide and N-(((2R,3S)-1-cyano-2-methylpyrrolidin-3-yl)methyl)-1-(3-cyanophenyl)-1H-1,2,4-triazole-3-carboxamide

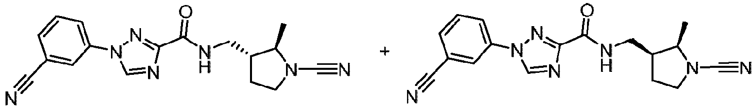

**Step a.** To a solution of methyl acetoacetate (200 g, 1724.13 mmol) and 1,2-dibromoethane (179.2 mL, 2068.95 mmol) in acetone (2000 mL) was added K₂CO₃ (356.8 g, 2586.19 mmol) at rt. The reaction mixture was heated at 70°C for 24 h. The resulting mixture was cooled to rt, filtered through celite and washed with acetone (2 x 100 mL). The filtrate was concentrated under reduced pressure to give a crude oil which was purified by column chromatography (4% EtOAc in hexane) to yield methyl 1-acetylcyclopropane-1-carboxylate (100 g, 704.22 mmol). LCMS: Method C, 1.47 min, MS: ES+ 143.14.

**Step b.** A solution of methyl 1-acetylcyclopropane-1-carboxylate (82.0 g, 577.46 mmol) and (R)-1-phenylethan-1-amine (69.87 g, 577.46 mmol) in toluene (820 mL) was charged in a dean stark glass assembly. The reaction mixture was heated to 130°C for 24 h (water release was collected in dean stark assembly). The reaction mixture was cooled to rt and concentrated under reduced pressure. The resulting residue was purified by column chromatography (1.5 % EtOAc in hexane) to yield methyl (R)-2-methyl-1-(1-phenylethyl)-4,5-dihydro-1H-pyrrole-3-carboxylate (51.0 g, 208.16 mmol). LCMS: Method C, 1.409 min, MS: ES+ 246.40.

**Step c.** To NaBH₄ (35.5 g, 936.73 mmol) was carefully added acetic acid (765 mL, 15 vol) dropwise at 0 °C and stirred for 45 min at 0°C. A solution of methyl (R)-2-methyl-1-(1-phenylethyl)-4,5-dihydro-1H-pyrrole-3-carboxylate (51.0 g, 208.16 mmol) in acetonitrile (765 mL) was then added at 0°C and the reaction mixture was stirred for 4 h at rt. The resulting mixture was diluted with water (4000 mL) and basified with solid Na₂CO₃. The aqueous layer was extracted with EtOAc (2 x 3000 mL). The combined organic phase was collected, dried over Na₂SO₄, filtered and concentrated under reduced pressure. The resulting residue was dissolved in hexane and insoluble solid was removed by filtration. The filtrate was evaporated under reduced pressure to yield a mixture of diastereomers (51.0 g, 208.16 mmol). LCMS: Method D-NH₃, 28.96 min (92%, major diasteromer) and 29.114 min (7%, minor diastereomer), MS: ES+ 248.2; The diastereomeric mixture was dissolved in n-hexane (500 mL) to form a clear solution which was stirred at -78 °C for 2 h. The resulting precipitate was removed by filtration and wash with cold hexane to yield methyl (2R,3R)-2-methyl-1-((R)-1-phenylethyl)pyrrolidine-3-carboxylate (28 g, 113.36 mmol). LCMS : Method D-NH₃, 28.819 min, Method D-FA: 12.098 min, MS: ES+ 248.2; ¹H NMR (400 MHz, DMSO-d₆) δ ppm: 7.29 - 7.35 (m, 4H), 7.22 - 7.24 (m, 1 H), 3.60 (s, 3 H), 3.55 - 3.58 (m, 1 H), 3.34-3.37 (m, 1H) 3.06 - 3.09 (m, 1 H), 2.58 - 2.61 (m, 1 H), 2.43 - 2.47 (m, 1 H), 1.92 - 1.99 (m, 1 H), 1.80 - 1.83 (m, 1 H), 1.27 (d, J=6.71 Hz, 3 H), 0.71 (d, J=6.40 Hz, 3 H).

### Step d.

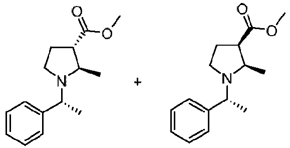

Methyl (2R,3R)-2-methyl-1-((R)-1-phenylethyl)pyrrolidine-3-carboxylate (26.0 g, 105.263 mmol) was dissolved in DBU (47.99 g, 315.723 mmol) and heated at 100°C for 24 h in seal tube. The reaction mixture was poured into water (800 mL) and extracted ethyl acetate with (3 x 700 mL). The combined organic layer was washed with water (100 mL), dried over Na₂SO₄ and evaporated under reduced pressure to give crude product which was purified by column chromatography (eluted at 30 % ethyl acetate in hexane ) to yield mixture of methyl (2R,3R)-2-methyl-1-((R)-1-phenylethyl)pyrrolidine-3-carboxylate and methyl (2R, 3S)-2-methyl-1-((R)-1-phenylethyl)pyrrolidine-3-carboxylate (22 g, 89.068 mmol). LCMS: Method D-FA, 11.94 min (21.72 %) & 12.68 min (77.01 %), MS: ES+ 248.4; ¹H NMR (400 MHz, CDCl₃) δ ppm: 7.25-7.40 (m, 5H), 3.85-3.87 (m, 1H), 3.74 (s, 3H), 3.00-3.30 (m, 1H), 2.56-2.81 (m, 3H), 1.93-2.08 (m, 2H), 1.41 (m, 3H), 1.04 (dd, J=6.4 Hz, 3H).

### Step e.

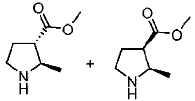

To solution of methyl (2R,3S)-2-methyl-1-((R)-1-phenylethyl) pyrrolidine-3-carboxylate and methyl (2R,3R)-2-methyl-1-((R)-1-phenylethyl)pyrrolidine-3-carboxylate (10 g, 40.485 mmol) in methanol (100 mL) was added 1 g (10% Pd/C, 50 % moisture). The reaction mixture was stirred at rt under hydrogen (40 barr) for 24 h. The resulting mixture was filtered through celit, washed with methanol (50mL) and the filtrate was concentrated under reduced pressure to yield mixture of methyl (2R,3S)-2-methylpyrrolidine-3-carboxylate and methyl (2R,3R)-2-methylpyrrolidine-3-carboxylate (5.3 g, 37.062 mmol). LCMS: Method C, 0.31 min, MS: ES+ 144.1; ¹H NMR (400 MHz, CDCl₃) δ ppm: 3.74 (s, 3H), 3.40-3.43 (m, 1H), 3.10-3.20 (m, 2H), 2.53-2.63 (m, 1H), 2.13-2.19 (m, 2H), 2.04-2.08 (m, 1H), 1.37 (dd, J=6.4 Hz, 3H).

### Step f.

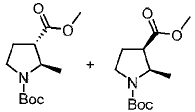

To solution of methyl (2R,3S)-2-methylpyrrolidine-3-carboxylate and methyl (2R,3R)-2-methylpyrrolidine-3-carboxylate (5.3 g, 37.062 mmol) in THF (53 mL) were added 4-dimethylaminopyridine (0.53 g, 0.1 w/w) and di-tert-butyl dicarbonate (9.69 g, 44.449 mmol) at 0°C under N₂. The reaction mixture was allowed to warm to rt and stirred for a further 16 h. The resulting mixture was diluted with water (200 mL) and extracted with ethyl acetate (3 x 200 mL).The combined organic layer was washed with water (200 mL), dried over Na₂SO₄ and evaporated under reduced pressure to give a crude product which was purified by column chromatography (eluted at 10% ethyl acetate in hexane ) to yield mixture of 1-(tert-butyl) 3-methyl (2R, 3S)-2-methylpyrrolidine-1,3-dicarboxylate and 1-(tert-butyl) 3-methyl (2R, 3R)-2-methylpyrrolidine-1,3-dicarboxylate (8.0g, 32.92 mmol). LCMS: Method C, 1.665 min, MS: ES+ (-56) 188 ; ¹H NMR (400 MHz, CDCl₃) δ ppm: 4.00-4.30 (m, 1H), 3.74 (s, 3H), 3.52-3.56 (m, 1H), 3.35-3.39 (m, 1H), 2.71-2.72 (m, 1H), 2.11-2.16 (m, 2H), 1.50 (s, 9H), 1.32 (dd, J=5.6 Hz, 3H).

### Step g.

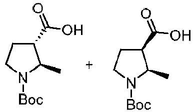

To a solution of 1-(tert-butyl) 3-methyl (2R,3S)-2-methylpyrrolidine-1,3-dicarboxylate and 1-(tert-butyl)-3-methyl (2R,3R)-2-methylpyrrolidine-1,3-dicarboxylate (8.0 g, 32.921 mmol) in THF: Water (5:1) (12 mL) was added lithium hydroxide monohydrate (2.07 g, 49.381 mmol) at 0°C. The resulting reaction mixture was warmed to rt and stirred for 6 h. The mixture was poured into water (300 mL) and washed with ethyl acetate (3 x 300 mL). The aqueous layer was acidified with 1M HCl solution (~200 mL) and extracted with ethyl acetate (3 x 300 mL). The combined organic layer was dried over Na₂SO₄ and evaporated under reduced pressure to yield mixture of (2R, 3R)-1-(tert-butoxycarbonyl)-2-methylpyrrolidine-3-carboxylic acid and (2R,3S)-(tert-butoxycarbonyl)-2-methylpyrrolidine-3-carboxylic acid (6.5 g, 28.384 mmol). LCMS: Method D-AA, 8.770 min (18.63 mmol) and 9.105 min (81.37 min), MS: ES+(-56) 156.25; ¹H NMR (400 MHz, DMSO-d₆) δ ppm: 12.47 (s, 1H), 3.8 - 4.1 (m, 1H), 3.25-3.37 (m, 2H), 2.60-2.75(m, 1H), 2.05-2.12 (m, 1H), 1.89-1.97 (m, 1H), 1.40 (s, 9H), 1.18 (dd, J=5.6 Hz, 3H).

### Step h.

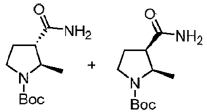

To a solution of (2R, 3R)-1-(tert-butoxycarbonyl)-2-methylpyrrolidine-3-carboxylic acid and (2R,3S)-1-(tert-butoxycarbonyl)-2-methylpyrrolidine-3-carboxylic acid (5.5 g, 24.017 mmol) in THF (50 mL) were added EDC.HCl (5.50 g, 28.795 mmol) and 1-hydroxybenzotriazole hydrate (3.67g, 23.986 mmol) at 0°C under N₂. Triethylamine (16.16 mL, 120.087 mmol) and NH₄Cl (6.42g, 120.000 mmol) were added to the reaction mixture and stirred for 16 h at rt. The resulting reaction mixture was poured into saturated NaHCO₃ solution (400 mL) and extracted with ethyl acetate (3 x 400 mL). The combined organic layer was dried over Na₂SO₄ and evaporated under reduced pressure to give crude product which was purified by column chromatography (2 % MeOH in DCM) to yield tert-butyl (2R,3R)-3-carbamoyl-2-methylpyrrolidine-1-carboxylate and tert-butyl (2R,3S)-3-carbamoyl-2-methylpyrrolidine-1-carboxylate (4.8 g, 21.056 mmol). LCMS: Method C, 1.380 min, MS: ES+ (-56) 173.2 ; ¹H NMR (400 MHz, CDCl₃) δ ppm: 5.57 (br, 2H), 3.95-4.12 (m, 1H), 3.42-3.63 (m, 2H), 2.42-2.61(m, 1H), 2.09-2.11 (m, 2H), 1.47 (s, 9H), 1.30 (dd, J=5.6 Hz, 3H).

### Step i.

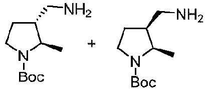

To a solution of tert-butyl (2R, 3R)-3-carbamoyl-2-methylpyrrolidine-1-carboxylate and tert-butyl (2R,3S)-3-carbamoyl-2-methylpyrrolidine-1-carboxylate (4.8 g, 21.052 mmol) in THF (20 mL) was added BH₃.DMS (2M in THF) (15.78 mL, 31.57 mmol) drop wise at 0 ⁰C under N₂. The reaction mixture was allowed to warm to rt and stirred for 16 h at 70 °C. The mixture was then cooled to rt and methanol (50 mL) added. The mixture was further heated at 80 °C for 2 h then cooled to rt, acidified with 1N HCl solution (240 mL) and extracted with ethyl acetate (3 x 400 mL). The aqueous layer was basified with sodium bicarbonate solution (50 mL) and extracted with ethyl acetate (3 x 300 mL) concentrated under reduced pressure to yield tert-butyl (2R,3R)-3-(amino methyl)-2-methylpyrrolidine-1-carboxylate and tert-butyl (2R,3S)-3-(amino methyl)-2-methylpyrrolidine-1-carboxylate (0.137 g, 0.640 mmol). LCMS: Method C, 1.322 min, MS: ES-56+ 159.31; ¹H NMR (400 MHz, DMSO-d₆) δ ppm: 3.28-3.48 (m, 3H), 3.15-3.26 (m, 1H), 2.41-2.44 (m, 1H), 1.65-2.05 (m, 2H), 1.41-1.60 (m, 3H), 1.34 (s, 9H), 0.95 (dd, J=6.4 Hz, 3H).

### Step j.

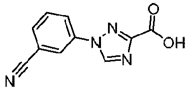

A stirred solution of methyl 1H-1,2,4-triazole-3-carboxylate (1.66 g, 13.1004 mmol) and 3-iodobenzonitrile (2 g, 8.733 mmol) in DMSO (10 mL) was added K₂CO₃ (3.6 g, 26.200 mmol) at rt. The reaction mixture was purged with N₂ for 15 min. L-proline (0.2 g, 1.746 mmol) and CuI (0.33 g, 1.746 mmol) were added at rt and the mixture was heated to 85^{°}C for 16 h. The mixture was then poured into water (200 mL) and extracted ethyl acetate (2 x 100 mL). The aqueous layer was acidified with 1M HCl solution (150 mL) and extracted with ethyl acetate (4 x 100 mL). The combined organic layer was washed with water (100 mL), dried over Na₂SO₄ and evaporated under reduced pressure to yield 1-(3-cyanophenyl)-1H-1,2,4-triazole-3-carboxylic acid (0.522 g, 63.11 mmol). LCMS: Method C, 1.29 min, MS: ES+ 215.23; ¹H NMR (400 MHz, DMSO-d₆) δ ppm: 12.8 (br, 1H), 7.35 (t, J=8 Hz, 1H), 7.02 (d, J=7.6 Hz, 1H), 6.83 (s, 1H), 6.77-6.80 (dd, J=8.4Hz, 2H).

### Step k.

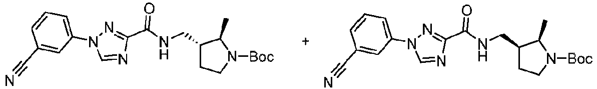

To a solution of 1-(3-cyanophenyl)-1H-1, 2, 4-triazole-3-carboxylic acid (0.156g, 0.728 mmol) in THF (7 mL) were added HATU (0.319 g, 0.841 mmol) and DIPEA (0.232 g, 1.682 mmol) 0°C under N₂. The reaction mixture was stirred for 30 min and added tert-butyl (2R, 3R)-3-(amino methyl)-2-methylpyrrolidine-1-carboxylate and tert-butyl (2R, 3S)-3-(amino methyl)-2-methylpyrrolidine-1-carboxylate (0.120 g, 0.560 mmol). The reaction mixture was become clear yellow solution and stirred at rt for 16 h. To resulting reaction mixture was added water (100 mL) and extracted with ethyl acetate (3 x 25 mL). The combined organic layer was washed with saturated NaHCO₃ solution (100 mL), dried over Na₂SO₄ and evaporated under reduced pressure to give crude product which was purified by Combi-flash chromatography (eluted at 0.2% MeOH in DCM) to yield tert-butyl (2R, 3R)-3-((1-(3-cyanophenyl)-1H-1, 2, 4-triazole-3-carboxamido) methyl)-2-methylpyrrolidine-1-carboxylate and tert-butyl (2R, 3S)-3-((1-(3-cyanophenyl)-1H-1, 2, 4-triazole-3-carboxamido) methyl)-2-methylpyrrolidine-1-carboxylate (0.106 g, 0.258 mmol). LCMS: Method C, 1.627 min, MS: ES+(-100) 311.48.

### Step l.

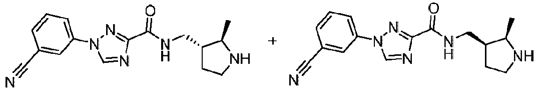

To a solution of tert-butyl (2R, 3S)-3-((1-(3-cyanophenyl)-1H-1, 2, 4-triazole-3-carboxamido) methyl)-2-methylpyrrolidine-1-carboxylate and tert-butyl (2R, 3R)-3-((1-(3-cyanophenyl)-1H-1, 2, 4-triazole-3-carboxamido) methyl)-2-methylpyrrolidine-1-carboxylate (0.103 g, 0.251 mmol) in DCM (5 mL) was added TFA (0.2 mL, 2.512 mmol) 0 ^{°}C under N₂ atmosphere. The reaction mixture was allowed to warm to rt and stirred for 2 h then concentrated under reduced pressure to yield 1-(3-cyanophenyl)-N-(((2R, 3R)-2-methylpyrrolidin-3-yl) methyl)-1H-1, 2, 4-triazole-3-carboxamide trifluoroacetate and 1-(3-cyanophenyl)-N-(((2R, 3S)-2-methylpyrrolidin-3-yl) methyl)-1H-1, 2, 4-triazole-3-carboxamide trifluoroacetate (0.140 g, 0.267 mmol). LCMS: Method C, 1.286 min, MS: ES+ 311.5.

### Step m.

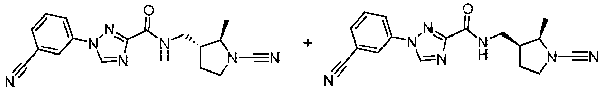

To a solution of 1-(3-cyanophenyl)-N-(((2R, 3S)-2-methylpyrrolidin-3-yl) methyl)-1H-1, 2, 4-triazole-3-carboxamide trifluoroacetate and 1-(3-cyanophenyl)-N-(((2R, 3R)-2-methylpyrrolidin-3-yl) methyl)-1H-1, 2, 4-triazole-3-carboxamide trifluoroacetate (0.139 g, 0.327 mmol) in THF (5mL) was added K₂CO₃ (0.090 g, 0.654 mmol) and the mixture was stirred for 10 min. CNBr (0.034 g, 0.392 mmol) was added at rt and reaction mixture was stirred at rt for 30 min. To resulting reaction mixture was added water (50 mL) and the mixture extracted with ethyl acetate (3 x 50 mL). The combined organic layer was washed with water (50 mL), dried over Na₂SO₄ and evaporated under reduced pressure to give crude product. The crude product was purified by Combi-flash chromatography (eluted at 0.4% MeOH in DCM) to yield N-(((2R, 3R)-1-cyano-2-methylpyrrolidin-3-yl) methyl)-1-(3-cyanophenyl)-1H-1, 2, 4-triazole-3-carboxamide (0.052g, 0.155 mmol) which was further purified by prep TLC (3% MeOH in DCM) and then prep HPLC (MeOH : ACN using 0.1% ammonia) then triturated with n-pentene (2 x 3 mL) to yield N-(((2R,3S)-1-cyano-2-methylpyrrolidin-3-yl)methyl)-1-(3-cyanophenyl)-1H-1,2,4-triazole-3-carboxamide and N-(((2R,3R)-1-cyano-2-methylpyrrolidin-3-yl)methyl)-1-(3-cyanophenyl)-1H-1,2,4-triazole-3-carboxamide (0.0028 g, 0.0083 mmol); LCMS: Method M, 17.82 (25.34 %),18.29 min (68.88 %), MS: ES+ 336; MS: ES+ 336.2.

The diastereoisomers may be separable by standard methods.

### Example 5

### (R)-1-(3-cyanophenyl)-N-((1-cyanopyrrolidin-3-yl)methyl)-1H-1,2,3-triazole-4-carboxamide

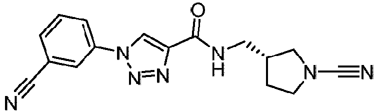

**Step a.** To a stirred solution of sodium azide (0.424 g, 6.535 mmol) and CuSO₄ (0.086 g, 0.544 mmol) in MeOH (8 mL) was added (3-cyanophenyl)boronic acid (0.8 g, 5.445 mmol) (CAS number 150255-96-2, available from Combi-Blocks) at rt. The reaction mixture was stirred at rt for 16 h. Ethyl propionate (0.587 g, 5.99 mmol) and a solution of sodium L-(+)-ascorbate (0.539 g, 0.272 mmol) in water (2 mL) was added to the reaction mixture which was stirred for a further 1 h at rt. The resulting reaction mixture was filtered, washed with water (3 x 5 mL) and the solid was dried under reduced pressure to yield ethyl 1-(3-cyanophenyl)-1H-1,2,3-triazole-4-carboxylate (0.25 g, 1.033 mmol). LCMS: Method C, 1.579 min; MS: ES+ 243.4.

**Step b.** To a stirred solution of ethyl 1-(3-cyanophenyl)-1*H*-1,2,3-triazole-4-carboxylate (0.2 g, 0.826 mmol) and tert-butyl (R)-3-(aminomethyl)pyrrolidine-1-carboxylate (0.165 g, 0.826 mmol) (CAS number 199174-29-3, available from Synthonix) in THF (5 mL) was added a solution of TBD (0.23 g, 1.653 mmol) in THF (1 mL) dropwise at 0 °C. The reaction mixture was heated at 80 °C for 16 h. The resulting reaction mixture was diluted with water (40 mL) and extracted with EtOAc (3 x 40 mL). The combined organic phase was dried over Na₂SO₄, filtered and concentrated under reduced pressure. Crude material was purified by flash column chromatography (69% EtOAc in hexane) to yield tert-butyl (*R*)-3-((1-(3-cyanophenyl)-1*H*-1,2,3-triazole-4-carboxamido)-methyl)-pyrrolidine-1-carboxylate (0.13 g, 0.328 mmol). LCMS: Method C, 1.659 min; MS: ES+397.48.

**Step c.** To a stirred solution of tert-butyl (*R*)-3-((1-(3-cyanophenyl)-1*H*-1,2,3-triazole-4-carboxamido)-methyl)-pyrrolidine-1-carboxylate (0.12 g, 0.303 mmol) in DCM (3 mL) was added TFA (1.2 mL, 10 volumes) dropwise at 0 °C and stirred at rt for 2 h. The resulting reaction mixture was concentrated under reduced pressure and the residue was further concentrated from DCM (3 x 5 mL) to yield (*S*)-1-(3-cyanophenyl)-*N*-(pyrrolidin-3-ylmethyl)-1*H*-1,2,3-triazole-4-carboxamide trifluoroacetate (0.14 g, 0.341 mmol). LCMS: Method C, 1.249 min; MS: ES+ 297.43.

**Step d.** To a stirred solution of (*S*)-1-(3-cyanophenyl)-*N*-(pyrrolidin-3-ylmethyl)-1*H*-1,2,3-triazole-4-carboxamide trifluoroacetate (0.13 g, 0.317 mmol) in THF (4 mL) was added K₂CO₃ (0.131 g, 0.951 mmol) at 0°C and was stirred for 10 min. Cyanogen bromide (0.04 g, 0.380 mmol) was added portionwise at 0 °C and stirred at rt for 1.5 h. The resulting reaction mixture was diluted with water (30 mL) extracted with EtOAc (3 x 30 mL). The combined organic phase was dried over Na₂SO₄, filtered and concentrated under reduced pressure. Crude material was purified by flash column chromatography (72% EtOAc in hexane) to yield (*R*)-1-(3-cyanophenyl)-*N*-((1-cyanopyrrolidin-3-yl) methyl)-1H-1,2,3-triazole-4-carboxamide (0.12 g, 0.374 mmol). LCMS: Method H, 2.97 min, MS: ES- 320.2; ¹H NMR (400 MHz, CDCl₃) δ ppm: 8.61 (s, 1 H), 8.18 (s, 1 H), 8.05 (m, 1 H), 7.86 - 7.75 (m, 2 H), 7.41 - 7.36 (m, 1 H), 3.65 - 3.47 (m, 4 H), 3.31 - 3.27 (m, 1 H), 2.71 - 2.64 (m, 1H), 2.22 - 2.12 (m, 1 H), 1.87 -1.78 (m, 1 H). Chiral HPLC: 100 % purity, retention time: 5.67 min

CHIRAL SFC Analytical Method for title compound:
Chiral compound was analysed on Waters SFC Investigator and PDA (Photodiode array) detector. The column was used Chiralcel OX-H 250*4.6 mm, 5 micron, column flow was 4.0 mL /min and ABPR (automated back-pressure regulator) was set to 100 bar. Mobile phase were used (A) liquid carbon dioxide (Liq. CO2) and (B) 0.1% diethylamine in propan-2-ol : acetonitrile (50:50).

The UV spectra were recorded at 246nm Lambda max. Gradient ratio was, as described below.

| %B Start | %B End | Time duration (min) |
|---|---|---|
| 5 | 50 | 5 |
| 50 | 50 | 5 |

### Example 6

### (R)-1-(3-chlorophenyl)-N-((1-cyanopyrrolidin-3-yl)methyl)-1H-1,2,3-triazole-4-carboxamide

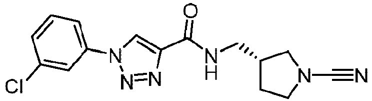

**Step a.** To a stirred solution of 1-(3-chlorophenyl)-1*H*-1,2,3-triazole-4-carboxylic acid (0.15 g, 0.671 mmol) (CAS Number 944901-58-0, available from Synthonix) and tert-butyl (*R*)-3-(aminomethyl)pyrrolidine-1-carboxylate (0.134 g, 0.671 mmol) (CAS number 199174-29-3, available from Astatech) in pyridine (5 mL) was added POCl₃ (0.307 g, 0.19 mL, 2.013 mmol) dropwise at 0 °C and stirred at 0 °C for 40 min. The resulting reaction mixture was poured into cold water (30 mL) and extracted with EtOAc (3 x 30 mL). The combined organic phase was dried over Na₂SO₄, filtered and concentrated under reduced pressure. The residue was purified by flash column chromatography (70% EtOAc in hexane) to yield tert-butyl-(*R*)-3-((1-(3-chlorophenyl)-1*H-*1,2,3-triazole-4-carboxamido)-methyl)-pyrrolidine-1-carboxylate (0.235 g, 0.580 mmol). LCMS: Method C, 1.734 min; MS: ES+ 406.49.

**Step b.** To a stirred solution of tert-butyl (*R*)-3-((1-(3-chlorophenyl)-1*H*-1,2,3-triazole-4-carboxamido)-methyl)-pyrrolidine-1-carboxylate (0.23 g, 0.568 mmol) in DCM (5 mL) was added TFA (2.3 mL, 10 volumes) dropwise at 0 °C and stirred at rt for 1.5 h. The resulting reaction mixture was concentrated under reduced pressure and crude was further concentrated from DCM (3 x 5 mL) to yield (*S*)-1-(3-chlorophenyl)-*N*-(pyrrolidin-3-ylmethyl)-1*H*-1,2,3-triazole-4-carboxamide trifluoroacetate (0.35 g, 0.835 mmol). LCMS: Method C, 1.341 min, MS: ES+ 306.36.

**Step c.** To a stirred solution of (*S*)-1-(3-chlorophenyl)-*N*-(pyrrolidin-3-ylmethyl)-1*H*-1,2,3-triazole-4-carboxamide trifluoroacetate (0.34 g, 0.811 mmol) in THF (7 mL) was added K₂CO₃ (0.335 g, 2.434mmol) at 0 °C and stirred at 0 °C for 10 min. Cyanogen bromide (0.103 g, 0.974 mmol) was added portion wise to the reaction mixture. The reaction mixture was stirred at rt for 1.5h. The resulting reaction mixture was diluted with water (50 mL) extracted with EtOAc (3 x 50mL). The combined organic phase was dried over Na₂SO₄, filtered and concentrated under reduced pressure. The residue was purified by flash column chromatography (80% EtOAc in hexane) to yield (*R*)-1-(3-chlorophenyl)-*N*-((1-cyanopyrrolidin-3-yl)-methyl)-1H-1,2,3-triazole-4-carboxamide (0.13 g, 0.394 mmol). LCMS: Method H, 3.59 min; MS: ES+ 331.10; ¹H NMR (400MHz, DMSO-d6) δ ppm: 9.38 (s, 1 H), 8.97 - 8.94 (m, 1H), 8.14(s, 1H), 8.01 - 7.99 (d, J= 8.0 Hz, 1 H), 7.69 - 7.61 (m, 2 H), 3.48 - 3.19 (m, 7 H), 2.00 - 1.92 (m, 1 H), 1.75 - 1.66 (m, 1 H). Chiral HPLC (method as previously shown): 100 % purity, retention time: 4.42 min

### Example 7

### (R)-2-(3-chlorophenyl)-N-((1-cyanopyrrolidin-3-yl)methyl)-2H-1,2,3-triazole-4-carboxamide

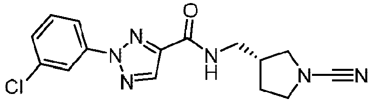

**Step a.** To a stirred solution of 2-(3-chlorophenyl)-2*H*-1,2,3-triazole-4-carboxylic acid (0.15 g, 0.671 mmol) (CAS Number 90839-69-3, available from Enamine) and tert-butyl (*R*)-3-(aminomethyl)pyrrolidine-1-carboxylate (0.134 g, 0.671 mmol) (CAS number 199174-29-3, available from Astatech) in pyridine (3 mL) was added POCl₃ (0.307 g, 0.19 mL, 2.013 mmol) dropwise at 0 °C and the mixture was stirred for 30 min. The resulting reaction mixture was poured in to water (100 mL) and extracted with EtOAc (3 x 50 mL). The combined organic phase was washed with water (100 mL), dried over Na₂SO₄, filtered and concentrated under reduced pressure. The crude material was purified by flash column chromatography (42% EtOAc in hexane) to yield tert-butyl (*R*)-3-((2-(3-chlorophenyl)-2*H*-1,2,3-triazole-4-carboxamido)-methyl)-pyrrolidine-1-carboxylate (0.175 g, 0.432 mmol). LCMS: Method C, 2.014 min; MS: ES+350.38 (M-56).

**Step b.** To a stirred solution of tert-butyl (*R*)-3-((2-(3-chlorophenyl)-2*H*-1,2,3-triazole-4-carboxamido)-methyl)-pyrrolidine-1-carboxylate (0.17 g, 0.419 mmol) in DCM (10 mL) was added TFA (0.478 g, 0.3 mL, 4.197 mmol) dropwise at 0 °C. The reaction mixture was stirred at rt for 2h. The resulting reaction mixture was concentrated under reduced pressure and crude was further concentrated from DCM (3 x 5 mL) to yield (*S*)-2-(3-chlorophenyl)-*N*-(pyrrolidin-3-ylmethyl)-2*H-*1,2,3-triazole-4-carboxamide trifluoroacetate (0.339 g, 0.809 mmol). LCMS: Method C, 1.374 min; MS: ES+306.31.

**Step c.** A solution of (*S*)-2-(3-chlorophenyl)-*N*-(pyrrolidin-3-ylmethyl)-2*H*-1,2,3-triazole-4-carboxamide trifluoroacetate (0.335 g, 0.799 mmol) in THF (10 mL) was added K₂CO₃ (0.22 g, 1.599 mmol) and the mixture was stirred for 10 min at 0 °C. Cyanogen bromide (0.102 g, 0.959 mmol) was added portionwise in to the reaction mixture and stirred at rt for 45 min. The resulting reaction mixture was diluted with water (50 mL) extracted with EtOAc (3 x 25 mL). The combined organic phase was washed with water (50 mL), dried over Na₂SO₄, filtered and concentrated under reduced pressure. The residue was purified by flash column chromatography (45% EtOAc in hexane) and by trituration with n-hexane (2 x 15 mL) to yield (*R*)-2-(3-chlorophenyl)-*N*-((1-cyanopyrrolidin-3-yl)-methyl)-2*H*-1,2,3-triazole-4-carboxamide (0.094 g, 0.284 mmol). LCMS: Method J, 3.99 min, MS: ES+ 331.4; ¹H NMR (400 MHz, CDCl₃) δ ppm: 8.31 (s, 1 H), 8.17 (s, 1H), 8.05 - 8.03 (m, 1H), 7.52 - 7.44 (m, 2 H), 7.08 - 6.99 (m, 1 H), 3.65 - 3.47 (m, 5 H), 3.32 - 3.27 (m, 1 H), 2.73 - 2.66 (m, 1 H), 2.20 - 2.13 (m, 1 H), 1.88 - 1.79 (m, 1 H). Chiral HPLC: 100 % purity, retention time: 14.05 min. Chiral compound was analysed on Agillent 1200 series HPLC and PDA detector. The column was used Chiralcel OJ-H 250*4.6 mm, 5 micron, column flow was 1.0 mL /min . Mobile phase were used (A) 0.1% diethylamine in hexane and (B) 0.1% diethylamine in propan-2-ol : methanol (50:50). The UV spectra were recorded at 272nm Lambda max. Gradient ratio was, as described below.

| Time | % A | % B |
|---|---|---|
| 0.01 | 90 | 10 |
| 5 | 85 | 15 |
| 10 | 65 | 35 |
| 15 | 45 | 55 |
| 20 | 35 | 65 |
| 25 | 35 | 65 |
| 25.01 | 90 | 10 |
| 30 | 90 | 10 |

### Example 8

### (R)-N-((1-cyanopyrrolidin-3-yl)methyl)-1-phenyl-1H-1,2,4-triazole-3-carboxamide

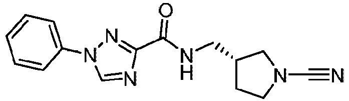

May be prepared using analogous procedures to those described herein.

### Biological Activity of Compounds of the Invention

### Abbreviations:

- TAMRA: carboxytetramethylrhodamine
- PCR: polymerase chain reaction
- PBS: phosphate buffered saline
- EDTA: ethylenediaminetetraacetic acid
- Tris: 2-amino-2-(hydroxymethyl)-1,3-propanediol
- NP-40: Nonidet P-40, octylphenoxypolyethoxyethanol
- BSA: bovine serum albumin
- PNS: peripheral nervous system
- BH3: Bcl-2 homology domain 3
- PTEN: phosphatase and tensin homologue

### USP30 biochemical IC50 assay

Dilution plates were prepared at 21 times the final concentration (2100µM for a final concentration of 100µM) in 50% DMSO in a 96-well polypropylene V-bottom plate (Greiner #651201). A typical 8-point dilution series would be 100, 30, 10, 3, 1, 0.3, 0.1, 0.03µM final. Reactions were performed in duplicate in black 384 well plates (small volume, Greiner 784076) in a final reaction volume of 21 µl. Either 1µl of 50% DMSO or diluted compound was added to the plate. USP30 was diluted in reaction buffer (40mM Tris, pH 7.5, 0.005% Tween 20, 0.5mg/ml BSA, 5 mM beta-mercaptoethanol) to the equivalent of 0.05µl/well and 10µl of diluted USP30 was added to the compound. Enzyme and compound were incubated for 30 min at room temp. Reactions were initiated by the addition of 50nM of TAMRA labelled peptide linked to ubiquitin via an iso-peptide bond as fluorescence polarisation substrate. Reactions were read immediately after addition of substrate and following a 2-hour incubation at room temperature. Readings were performed on a Pherastar Plus (BMG Labtech). λ Excitation 540 nm; λ Emission 590 nm.

### Activity of Exemplary Compounds in USP30 biochemical IC50 assay

### Ranges:

$A * < 0.01 μM ;$ $0.01 < A < 0.1 μM ;$ $0.1 < B < 1 μM ;$ $1 < C < 10 μM ;$ $D > 10 μM ;$

| Example | IC50 |
|---|---|
| 1 | A* |
| 2 | A |
| 3 | B |
| 4 | A |
| 5 | B |
| 6 | B |
| 7 | A* |

| Examples of WO 2017/103614 | | | | | | | |
|---|---|---|---|---|---|---|---|
| Example | IC50 | | Example | IC50 | | Example | IC50 |
| 9 | A | | 38 | B | | 67 | B |
| 10 | B | | 55 | A | | 68 | A* |
| 20 | B | | 58 | B | | 69 | A |
| 21 | B | | 61 | B | | 70 | C |
| 22 | B | | 62 | B | | 71 | A |
| 25 | B | | 63 | B | | 72 | B |
| 26 | B | | 64 | B | | 73 | B |
| 31 | B | | 66 | B | | 74 | B |

Compounds of the invention may be tested for efficacy in representative disease models, including *in vivo* models.

### MT#1: (R)-5-(3-cyanophenyl)-N-((1-cyanopyrrolidin-3-yl)methyl)-1,3,4-oxadiazole-2-carboxamide (WO 2017/103614: Ex. 68)

### Representative models and experimental results for MT#1:

### Bleomycin-induced lung fibrosis model

Bleomycin historically has been used in the treatment of lymphoma, squamous cell carcinomas, germ cell tumors and malignant pleural effusion, where it is injected intrapleurally. Bleomycin acts by causing single and double-strand DNA breaks in tumor cells and thereby interrupting the cell cycle. However, a concomitant overproduction of reactive oxygen species can lead to an inflammatory response causing pulmonary damage and subsequent fibrosis. Pulmonary side effects in patients are more likely in patients that smoke, are of older age, or have pre-existing pulmonary conditions. Dose dependent lung toxicity develops in approximately 10% of patients receiving bleomycin, and is clinically associated with cough, dyspnea, fever, cyanosis, and deterioration of lung function parameters. Within weeks to months this response might progress to pulmonary fibrosis in approximately 1% of patients.

Bleomycin as an agent to induce experimental lung fibrosis was first described in dogs later in mice, hamsters, and rats. It causes an initial elevation of pro-inflammatory cytokines, followed by increased expression of pro-fibrotic markers (transforming growth factor-β1, fibronectin, procollagen-1), with a peak around day 14. Fibrosis tends to dominate pathology from day 9 onwards. Bleomycin-induced lung fibrosis is currently the mainstay of preclinical in vivo modelling of Idiopathic Pulmonary Fibrosis in the pharmaceutical industry. Current standard of care drugs such as pirfenidone and nintedanib have been tested in this preclinical lung fibrosis model.

In this study, Male C57B/L6 mice between ages of 6 to 8 weeks were used for a dose ranging study with MT#1. Treatment group animals (n=10/group) received 1.5U/kg of clinical bleomycin via oropharyngeal route on day 0 under inhalant anaesthesia. Animals were monitored for tolerance of the bleomycin administration procedure and subsequently bodyweights of all mice were recorded at least 3 times per week during the study. Dosing with MT#1 or positive control (pirfenidone) commenced on day 7, with final dosing administered 2 to 4 hours prior to harvest on day 21 post bleomycin. All treatments were administered orally. MT#1 was supplied as a nanosuspension prepared at 100mg/ml in hydroxymethylpropylcellulose/PVP/SDS (0.5%/0.5%/0.1%).

The following table summarizes treatment groups:

| Treatment Group | Bleomycin | Treatment (days 7 to 21) | Test article daily dosing volume | Number of animals per group | In life mortality |
|---|---|---|---|---|---|
| 1 | 0 U/kg | None | - | 10 | 0% |
| 2 | 1.5 U/kg | Vehicle BID | 200µl | 10 | 0% |
| 3 | 1.5 U/kg | 50mg/kg MT#1 nanosuspension BID | 200µl | 10 | 0% |
| 4 | 1.5 U/kg | 15mg/kg MT#1 nanosuspension BID | 200µl | 10 | 10% |
| 5 | 1.5 U/kg | 5mg/kg MT#1 nanosuspension BID | 200µl | 10 | 10% |
| 6 | 1.5 U/kg | 100mg/kg pirfenidone BID | 200µl | 10 | 0% |
| 7 | 1.5 U/kg | 15mg/kg MT#1 nanosuspension BID + 100mg/kg Pirfenidone BID | 400µl | 10 | 10% |

At day 21, lungs were harvested from each animal and weighed, followed by fixation in 10% neutral buffered formalin (NBF) for histopathological analysis. The lung samples were processed and embedded with all lobes from each mouse in one paraffin block. Coronal sections through the four major lobes were stained with Masson's Trichrome. For each animal, consecutive lung fields were examined at 200X magnification in a raster pattern using a 20X objective lens and a 10X ocular lens. A modified Ashcroft score (Hubner et al., 2008) was recorded for each field. The Fibrotic Index was calculated as the sum of the modified Ashcroft field scores divided by the number of fields examined.

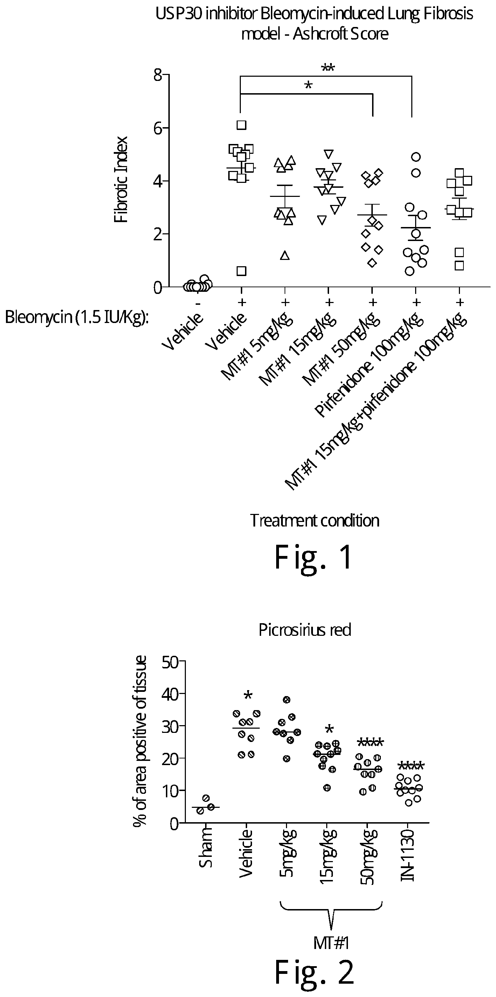

Figure 1. Effects of MT#1 treatment on Ashcroft Score in Bleomycin-induced lung fibrosis.

Treatment with MT#1 resulted in a dose dependent reduction in fibrosis as assessed by the modified Ashcroft score (collagen deposition, septae thickening). A statistically significant effect compared to vehicle was observed at 50mg/kg BID (Statistics = one-way ANOVA + Tukey post-testing; * - p<0.05, ** - p<0.01).

### Unilateral ureteral obstructive kidney disease model (UUO)

Unilateral ureteral obstruction (UUO) causes renal injury characterized by tubular cell injury, interstitial inflammation and fibrosis. It serves as a model both of irreversible acute kidney injury and as a model of events taking place during human chronic kidney disease. Being a unilateral disease, it is not useful to study changes in global kidney function, but has the advantage of a low mortality and the availability of an internal control (the non-obstructed kidney). Experimental UUO has illustrated the molecular mechanisms of apoptosis, inflammation and fibrosis, all three key processes in kidney injury of any cause, thus providing information beyond obstruction. The UUO model is commonly used to study drugs for potential therapeutic benefit in kidney fibrosis, including TGF-beta receptor inhibitors (galunisertib; LY2157299) and anti-TGF-beta antibodies (fresolumimab).

In this study, adult C57Bl6 female mice at 7 to 8 weeks of age with average weight of around 18 to 20g were used. To complete the ureteral obstruction, the abdominal cavity was exposed *via* a midline incision and the left ureter was ligated at two points with 4-0 silk and dissected. Successful unilateral ureteral obstruction was later confirmed by observation of dilation of the renal pelvis due to hydronephrosis.

The following table summarizes treatment groups:

| Treatment Group | Surgery | Treatment (days 0 to 10) | Test article Administration route (PO-oral, IP - intraperitoneal) | Number of animals per group | In life mortality | Number of completed animals excluded from analysis |
|---|---|---|---|---|---|---|
| 1 | Sham | None | - | 3 | 0% | 0 |
| 2 | UUO | Vehicle BID | PO | 10 | 20% | 0 |
| 3 | UUO | 5mg/kg MT# 1 nanosuspension BID | PO | 10 | 10% | 1 |
| 4 | UUO | 15mg/kg MT#1 nanosuspension BID | PO | 10 | 0% | 0 |
| 5 | UUO | 50mg/kg MT#1 nanosuspension BID | PO | 10 | 10% | 0 |
| 6 | UUO | 20mg/kg IN-1130 QD | IP | 10 | 0% | 0 |

At harvest, mice were anesthetised and both kidneys were harvested; the contralateral kidney serves as control for the UUO kidney. The kidneys were dissected along median line into two parts, of which one side was fixed in formaldehyde, for paraffin embedding and the other was snap frozen for protein or RNA isolation.

Formalin fixed tissue were embedded in paraffin blocks and cut into 5µm sections. All damaged kidneys, 5 contralateral kidneys from each group and 3 sham kidneys were analysed (in total n=80 kidney samples). Picrosirius Red and α-SMA stain was performed to evaluate fibrotic changes and myofibroblast activation. Analysis was performed by a pathology trained technical staff member, without knowledge of the animal group assignment. High magnification images were taken of each slide an analysed for the surface area covered by the stain.

Figure 2. Effects of MT#1 treatment in UUO model.

Treatment with MT#1 resulted in a dose dependent decrease in collagen deposition as measured by picrosirius red staining. Statistically significant decreases in collagen were observed at 15 and 50 mg/kg BID MT#1.

### Diet-induced model of Non-alcoholic Fatty Liver Disease (NAFLD)/Non-Alcoholic Steatohepatitis (NASH)

Mice were fed Amylin-diet (D09100301, Research Diets, US; 40% fat (18% trans-fat), 40% carbohydrate (20% fructose) and 2% cholesterol) for 37 weeks. Liver biopies were harvested and mice were randomization into treatment groups based on liver Col1a1 quantification. Three weeks following biopsy surgery, study animals were dosed orally (P.O.) with either; Vehicle (0.5% HPMC/0.5% PVP/0.1% SDS nanosuspension), MT#1 @ 15 mg/kg QD or MT#1 @ 15 mg/kg QD staining or with Elafibranor @ 30 mg/kg QD (positive control) for eight (8) weeks. A control group of age- and sex-matched mice fed lean chow diet *ad libitum* were also administered vehicle (P.O) over the same 8 (eight) week period. Following the full dosing schedule, mice were sacrificed, whole livers resected and weighed, fixed in paraformaldehyde before being paraffin embedded. H&E stained slides were then prepared and the average % fractional area of lipid vacuoles (steatosis) determined for each section (@ 20 x objective) using Visiomorph software (Visophram, Denmark). The average % fractional area was multiplied by the total liver weight to determine the total liver lipid content per animal. Values expressed as mean of n = 9-12 + SEM. Dunnett's test one-factor linear model. * *: P < 0.01, ***: P < 0.001 compared to DIO-NASH Vehicle.

Effects of MT#1 treatment in NAFLD/NASH model.

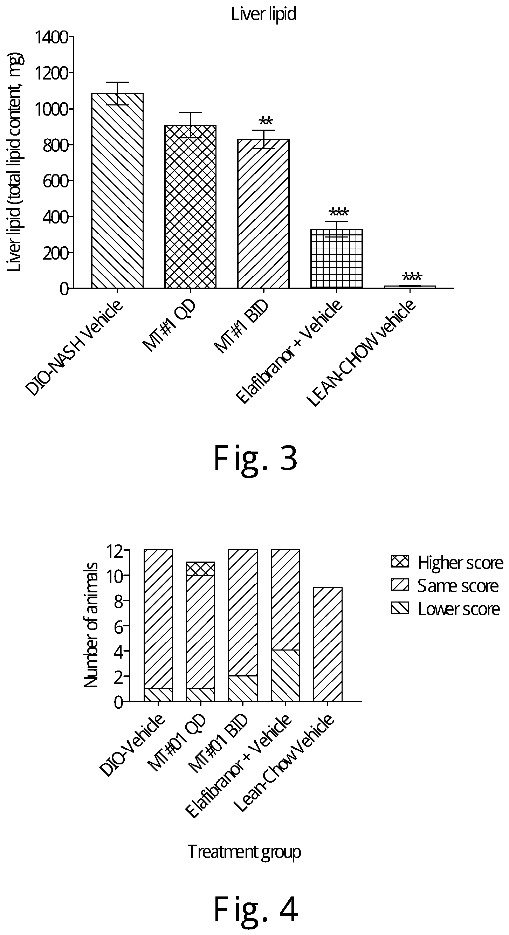

Figure 3. MT#1 administration resulted in a dose-dependent reduction in total liver lipid content in NASH animals, an effect that was statistically significant in mice administered MT#1 ~ 15 mg/kg BID (P.O.).

Figure 4. Neither MT#1 or Elafibranor (positive control) significantly improved fibrotic stage scores in the same diet-induced Nonalcoholic Fatty Liver Disease (NAFLD)/Nonalcoholic Steatohepatitis (NASH).

Summary of fibrosis stage scores of pre- and post-study liver biopsies harvested from the diet-induced NAFLD/NASH study. Fibrosis stage scores are determined by assessment of picosirius red stained liver sections for relative appearance and location of collagen staining, where; 0 = No staining, 1 = Perisinusoidal or periportal, 2 = Perisinusoidal and periportal and 4 = Bridging collagen staining observed. For each group of animals with higher (worsening), same or lower (improvement) in score at post- compared to pre-study is indicated by the height of the bar. One-sided Fisher's exact test with Bonferroni correction. No differences at significance level 0.05.

Despite eliciting a statistically significant reduction in liver steatosis, MT#1 did not significantly improve fibrotic stage scores in treated animals. However, in this particular study, Elafibranor, whilst significantly reducing liver steatosis also failed to statistically improve fibrosis scoring in post-biopsy samples. Hence, it is possible that a longer period of compound administration may have been required to see a robust effect on fibrosis in this particular preclinical model of NAFLD/NASH.

### Ischemia-induced acute kidney injury model (AKI)

Severe blood loss during major operations, sepsis and cardio-thoracic surgeries are common causes for acute kidney injury (AKI). The incidence of AKI after lung transplantation is 50 to 60% and leads to increased morbidity and mortality of those patients. Similar AKI rates are present in other types of solid organ transplantation (i.e. liver, heart). Underlying cellular mechanisms include rapid complement activation, generation of oxygen free radicals, up regulation of cell adhesion molecules and inflammatory cell infiltration. Long term consequences are progressive interstitial fibrosis and chronic kidney disease. AKI can be induced by bilateral renal pedical clamping resulting in ischemia reperfusion injury (IRI) resulting in severe loss of renal function tubular damage and inflammation.

12 to 15-week old male C57Bl/6N mice or CD-1 mice (Charles River, Germany) are used for the study. Under anaesthesia, a midline incision is performed and both renal pedicles are clipped with a microaneurysm clip for 30 min. Without treatment mice show an increase of creatinine within 24h of 3 to 6 fold and mortality can reach 30 to 40% with the first 4 days after surgery.

## Claims

1. A compound of formula (I): 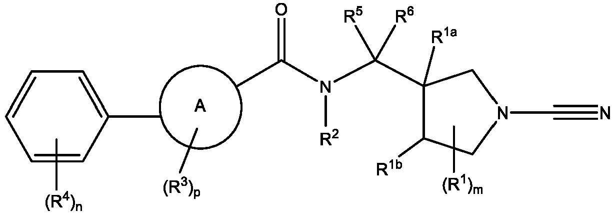 a tautomer thereof, or a pharmaceutically acceptable salt of said compound or tautomer, or a pharmaceutically acceptable composition comprising said compound or salt, for use in the treatment of fibrosis, wherein:
m is 0 to 5;
n is 0 to 5;
p is 0 to 3;
ring A is a monocyclic heteroaryl ring containing 1, 2 or 3 heteroatoms, each independently selected from N, O and S;
each R¹ is independently selected from halo, cyano, hydroxy, (C₁-C₆)alkyl, (C₁-C₆)alkoxy, halo(C₁-C₆)alkyl, halo(C₁-C₆)alkoxy, and (C₁-C₆)alkoxy(C₁-C₆)alkyl;
R^{1a} and R^{1b}, are each independently selected from hydrogen, halo, cyano, hydroxy, (C₁-C₆)alkyl, (C₁-C₆)alkoxy, halo(C₁-C₆)alkyl, halo(C₁-C₆)alkoxy, and (C₁-C₆)alkoxy(C₁-C₆)alkyl;
R² is selected from hydrogen, (C₁-C₆)alkyl, and (C₁-C₆)alkoxy(C₁-C₆)alkyl;
each R³ is independently selected from hydrogen, halo, cyano, hydroxy, (C₁-C₆)alkyl, (C₁-C₆)alkoxy, (C₁-C₆)alkoxy(C₁-C₆)alkyl, halo(C₁-C₆)alkyl, halo(C₁-C₆)alkoxy, NH(C₁-C₆)alkyl, N((C₁-C₆)alkyl)₂, C(O)NH(C₁-C₆)alkyl, C(O)N((C₁-C₆)alkyl)₂, NHC(O)(C₁-C₆)alkyl, N((C₁-C₆)alkyl)C(O)(C₁-C₆)alkyl), C(O)(C₁-C₆)alkyl, C(O)O(C₁-C₆)alkyl, CO₂H, CONH₂, SO₂NH(C₁-C₆)alkyl, and SO₂N((C₁-C₆)alkyl)₂;
each R⁴ is independently selected from halo, cyano, hydroxy, (C₁-C₆)alkyl, (C₁-C₆)alkoxy, (C₁-C₆)alkoxy(C₁-C₆)alkyl, halo(C₁-C₆)alkyl, halo(C₁-C₆)alkoxy, NH(C₁-C₆)alkyl, N((C₁-C₆)alkyl)₂, C(O)NH(C₁-C₆)alkyl, C(O)N((C₁-C₆)alkyl)₂, NHC(O)(C₁-C₆)alkyl, N((C₁-C₆)alkyl)C(O)(C₁-C₆)alkyl), C(O)(C₁-C₆)alkyl, C(O)O(C₁-C₆)alkyl, CO₂H, CONH₂, SO₂NH(C₁-C₆)alkyl, and SO₂N((C₁-C₆)alkyl)₂; and
R⁵ and R⁶ are each independently selected from hydrogen, cyano, and (C₁-C₆)alkyl; or R⁵ and R⁶ together form a 3 to 6 membered cycloalkyl ring.

2. A compound for use, according to claim 1, wherein m is 0 or 1; n is 0, 1 or 2; and p is 0 or 1.

3. A compound for use, according to either claim 1 or claim 2, wherein
R¹ is selected from chloro, fluoro, methyl, ethyl, and methoxy;
R^{1a} is selected from hydrogen, chloro, fluoro, cyano, hydroxy, methyl, ethyl, methoxy, and methoxymethyl; and
R^{1b} is selected from hydrogen, chloro, fluoro, cyano, hydroxy, methyl, ethyl, methoxy, and methoxymethyl.

4. A compound for use, according to any one of claims 1 to 3, wherein:
(i) m is 0, R^{1a} is hydrogen, and R^{1b} is methyl;
(ii) m is 1, R¹ is methyl, and R^{1a} and R^{1b} are each hydrogen; or
(iii) m is 0, and R^{1a} and R^{1b} are each hydrogen.

5. A compound for use, according to any one of claims 1 to 4, wherein
R² is selected from hydrogen and methyl;
each R³ is independently selected from hydrogen, chloro, fluoro, cyano, hydroxy, methyl, ethyl, propyl, methoxy, ethoxy, and methoxymethyl;
each R⁴ is independently selected from chloro, fluoro, cyano, hydroxy, methyl, ethyl, propyl, methoxy, ethoxy, methoxymethyl, CF₃, andOCF₃; and
R⁵ and R⁶ are each independently selected from hydrogen, cyano and methyl.

6. A compound for use, according to claim 1, which is selected from:
1-(3-cyanophenyl)-N-((1-cyanopyrrolidin-3-yl)methyl)-1H-1,2,4-triazole-3-carboxamide;
(R)-1-(3-cyanophenyl)-N-((1-cyanopyrrolidin-3-yl)methyl)-1H-1,2,4-triazole-3-carboxamide;
(S)-1-(3-cyanophenyl)-N-((1-cyanopyrrolidin-3-yl)methyl)-1H-1,2,4-triazole-3-carboxamide;
1-(3-cyanophenyl)-N-((1-cyanopyrrolidin-3-yl)methyl)-N-methyl-1H-1,2,4-triazole-3-carboxamide;
(R)-1-(3-cyanophenyl)-N-((1-cyanopyrrolidin-3-yl)methyl)-N-methyl-1H-1,2,4-triazole-3-carboxamide;
(S)-1-(3-cyanophenyl)-N-((1-cyanopyrrolidin-3-yl)methyl)-N-methyl-1H-1,2,4-triazole-3-carboxamide;
N-((1-cyanopyrrolidin-3-yl)methyl)-1-phenyl-1H-1,2,4-triazole-3-carboxamide;
(R)-N-((1-cyanopyrrolidin-3-yl)methyl)-1-phenyl-1H-1,2,4-triazole-3-carboxamide;
(S)-N-((1-cyanopyrrolidin-3-yl)methyl)-1-phenyl-1H-1,2,4-triazole-3-carboxamide;
N-(((2S,3S)-1-cyano-2-methylpyrrolidin-3-yl)methyl)-1-(3-cyanophenyl)-1H-1,2,4-triazole-3-carboxamide;
N-(((2S,3R)-1-cyano-2-methylpyrrolidin-3-yl)methyl)-1-(3-cyanophenyl)-1H-1,2,4-triazole-3-carboxamide;
N-(((2R)-1-cyano-2-methylpyrrolidin-3-yl)methyl)-1-(3-cyanophenyl)-1H-1,2,4-triazole-3-carboxamide;
N-(((2R,3R)-1-cyano-2-methylpyrrolidin-3-yl)methyl)-1-(3-cyanophenyl)-1H-1,2,4-triazole-3-carboxamide;
N-(((2R,3S)-1-cyano-2-methylpyrrolidin-3-yl)methyl)-1-(3-cyanophenyl)-1H-1,2,4-triazole-3-carboxamide;
N-(((2S)-1-cyano-2-methylpyrrolidin-3-yl)methyl)-1-(3-cyanophenyl)-1H-1,2,4-triazole-3-carboxamide;
1-(3-cyanophenyl)-N-((1-cyanopyrrolidin-3-yl)methyl)-1H-1,2,3-triazole-4-carboxamide;
(R)-1-(3-cyanophenyl)-N-((1-cyanopyrrolidin-3-yl)methyl)-1H-1,2,3-triazole-4-carboxamide;
(S)-1-(3-cyanophenyl)-N-((1-cyanopyrrolidin-3-yl)methyl)-1H-1,2,3-triazole-4-carboxamide;
1-(3-chlorophenyl)-N-((1-cyanopyrrolidin-3-yl)methyl)-1H-1,2,3-triazole-4-carboxamide;
(R)-1-(3-chlorophenyl)-N-((1-cyanopyrrolidin-3-yl)methyl)-1H-1,2,3-triazole-4-carboxamide;
(S)-1-(3-chlorophenyl)-N-((1-cyanopyrrolidin-3-yl)methyl)-1H-1,2,3-triazole-4-carboxamide;
2-(3-chlorophenyl)-N-((1-cyanopyrrolidin-3-yl)methyl)-2H-1,2,3-triazole-4-carboxamide;
(R)-2-(3-chlorophenyl)-N-((1-cyanopyrrolidin-3-yl)methyl)-2H-1,2,3-triazole-4-carboxamide;
(S)-2-(3-chlorophenyl)-N-((1-cyanopyrrolidin-3-yl)methyl)-2H-1,2,3-triazole-4-carboxamide;
N-((1-cyanopyrrolidin-3-yl)methyl)-2-phenyloxazole-5-carboxamide;
N-((1-cyanopyrrolidin-3-yl)methyl)-3-phenylisoxazole-5-carboxamide;
N-((1-cyanopyrrolidin-3-yl)methyl)-5-phenyl-1H-pyrazole-3-carboxamide;
N-((1-cyanopyrrolidin-3-yl)methyl)-3-(o-tolyl)-1H-pyrazole-5-carboxamide;
N-((1-cyanopyrrolidin-3-yl)methyl)-2-phenylthiazole-4-carboxamide;
N-((1-cyanopyrrolidin-3-yl)methyl)-3-(2-fluorophenyl)-1H-pyrazole-5-carboxamide;
N-((1-cyanopyrrolidin-3-yl)methyl)-5-phenyloxazole-2-carboxamide;
(R)-3-(3-chlorophenyl)-N-((1-cyanopyrrolidin-3-yl)methyl)isoxazole-5-carboxamide;
(R)-N-((1-cyanopyrrolidin-3-yl)methyl)-5-phenylisoxazole-3-carboxamide;
(R)-N-((1-cyanopyrrolidin-3-yl)methyl)-5-phenylthiazole-2-carboxamide;
(R)-N-((1-cyanopyrrolidin-3-yl)methyl)-4-phenylthiazole-2-carboxamide;
(R)-N-((1-cyanopyrrolidin-3-yl)methyl)-1-phenyl-1H-pyrazole-3-carboxamide;
(R)-N-((1-cyanopyrrolidin-3-yl)methyl)-2-phenyl-1H-imidazole-5-carboxamide;
(R)-3-(2-chlorophenyl)-N-((1-cyanopyrrolidin-3-yl)methyl)isoxazole-5-carboxamide;
(R)-3-(4-chlorophenyl)-N-((1-cyanopyrrolidin-3-yl)methyl)isoxazole-5-carboxamide;
(R)-5-(3-cyanophenyl)-N-((1-cyanopyrrolidin-3-yl)methyl)-1,3,4-oxadiazole-2-carboxamide;
(S)-5-(3-cyanophenyl)-N-((1-cyanopyrrolidin-3-yl)methyl)-1,3,4-oxadiazole-2-carboxamide;
(R)-N-((1-cyanopyrrolidin-3-yl)methyl)-1-phenyl-1H-imidazole-4-carboxamide;
(R)-1-(3-cyanophenyl)-N-((1-cyanopyrrolidin-3-yl)methyl)-1H-imidazole-4-carboxamide;
(R)-1-(4-cyanophenyl)-N-((1-cyanopyrrolidin-3-yl)methyl)-1H-imidazole-4-carboxamide;
(R)-N-((1-cyanopyrrolidin-3-yl)methyl)-1-(2-methoxyphenyl)-1H-imidazole-4-carboxamide; and
(R)-N-((1-cyanopyrrolidin-3-yl)methyl)-1-(3-methoxyphenyl)-1H-imidazole-4-carboxamide;
a tautomer thereof, or a pharmaceutically acceptable salt of said compound or tautomer.

7. A compound that is a USP30 inhibitor, or a pharmaceutically acceptable composition comprising said compound, for use in the treatment of fibrosis.

8. A compound or composition according to any one of claims 1 to 7 for use in the oral treatment of fibrosis.

9. A compound or composition for use, according to any one of claims 1 to 8, wherein the treatment is prophylactic.

10. A compound or composition for use, according to any one of claims 1 to 9, wherein the fibrosis is selected from fibrosis or a fibrotic disorder associated with the accumulation of extracellular matrix constituents that occurs following trauma, inflammation, tissue repair, immunological reactions, cellular hyperplasia, and neoplasia.

11. A compound or composition for use, according to claim 10, wherein the fibrosis is selected from fibrosis or a fibrotic disorder associated with major organ diseases, fibroproliferative disorders, and scarring associated with trauma.

12. A compound or composition for use, according to claim 11, wherein the fibrosis is selected from fibrosis or a fibrotic disorder associated with interstitial lung disease, liver cirrhosis, non-alcoholic fatty liver disease, non-alcoholic steatohepatitis, kidney disease, heart or vascular disease, diseases of the eye, systemic and local scleroderma, keloids, hypertrophic scars, atherosclerosis, restenosis, Dupuytren's contracture, surgical complications, chemotherapeutics drug-induced fibrosis, radiation-induced fibrosis, accidental injury and burns, retroperitoneal fibrosis, and peritoneal fibrosis/peritoneal scarring.

13. A compound or composition for use, according to claim 12, wherein the fibrosis is selected from fibrosis or a fibrotic disorder associated with kidney disease.

14. A compound or composition for use, according to claim 13, wherein the fibrosis associated with interstitial lung disease is selected from sarcoidosis, silicosis, drug reactions, infections, collagen vascular diseases, rheumatoid arthritis, systemic sclerosis, scleroderma, pulmonary fibrosis, idiopathic pulmonary fibrosis, usual interstitial pneumonitis, interstitial lung disease, cryptogenic fibrosing alveolitis, bronchiolitis obliterans, and bronchiectasis.

15. A compound or composition for use, according to claim 12, wherein the fibrosis associated with liver cirrhosis is selected from cirrhosis associated with viral hepatitis, schistosomiasis and chronic alcoholism.

## Patentansprüche

1. Verbindung der Formel (I): 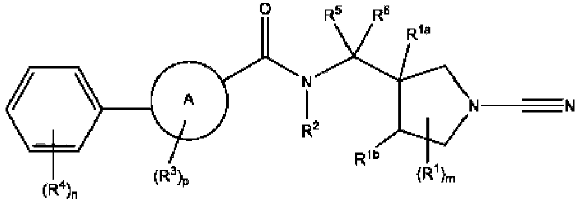 ein Tautomer davon oder ein pharmazeutisch unbedenkliches Salz der Verbindung oder des Tautomers oder eine pharmazeutisch unbedenkliche Zusammensetzung, umfassend die Verbindung oder das Salz, zur Verwendung bei der Behandlung von Fibrose, wobei:
m 0 bis 5 ist;
n 0 bis 5 ist;
p 0 bis 3 ist;
Ring A ein monocyclischer Heteroarylring ist, enthaltend 1, 2 oder 3 Heteroatome, die jeweils unabhängig aus N, O und S ausgewählt sind;
jedes R¹ unabhängig aus Halo, Cyano, Hydroxy, (C₁-C₆)-Alkyl, (C₁-C₆)-Alkoxy, Halo-(C₁-C₆)-alkyl, Halo-(C₁-C₆)-alkoxy und (C₁-C₆)-Alkoxy-(C₁-C₆)-alkyl ausgewählt ist;
R^{1a} und R^{1b} jeweils unabhängig aus Wasserstoff, Halo, Cyano, Hydroxy, (C₁-C₆)-Alkyl, (C₁-C₆)-Alkoxy, Halo-(C₁-C₆)-alkyl, Halo-(C₁-C₆)-alkoxy und (C₁-C₆)-Alkoxy-(C₁-C₆)-alkyl ausgewählt sind;
R² aus Wasserstoff, (C₁-C₆)-Alkyl und (C₁-C₆)-Alkoxy-(C₁-C₆)-alkyl ausgewählt ist;
jedes R³ unabhängig aus Wasserstoff, Halo, Cyano, Hydroxy, (C₁-C₆)-Alkyl, (C₁-C₆)-Alkoxy, (C₁-C₆)-Alkoxy-(C₁-C₆)-alkyl, Halo-(C₁-C₆)-alkyl, Halo-(C₁-C₆)-alkoxy, NH(C₁-C₆)-Alkyl, N((C₁-C₆)-Alkyl)₂, C(O)NH(C₁-C₆)-Alkyl, C(O)N((C₁-C₆)-Alkyl)₂, NHC(O)(C₁-C₆)-Alkyl, N((C₁-C₆)-Alkyl)-C(O)(C₁-C₆)-alkyl), C(O)(C₁-C₆)-Alkyl, C(O)O(C₁-C₆)-Alkyl, CO₂H, CONH₂, SO₂NH(C₁-C₆)-Alkyl und SO₂N((C₁-C₆)-Alkyl)₂ ausgewählt ist;
jedes R⁴ unabhängig aus Halo, Cyano, Hydroxy, (C₁-C₆)-Alkyl, (C₁-C₆)-Alkoxy, (C₁-C₆)-Alkoxy-(C₁-C₆)-alkyl, Halo-(C₁-C₆)-alkyl, Halo-(C₁-C₆)-alkoxy, NH(C₁-C₆)-Alkyl, N((C₁-C₆)-Alkyl)₂, C(O)NH(C₁-C₆)-Alkyl, C(O)N((C₁-C₆)-Alkyl)₂, NHC(O)(C₁-C₆)-Alkyl, N((C₁-C₆)-Alkyl)-C(O)(C₁-C₆)-alkyl), C(O)(C₁-C₆)-Alkyl, C(O)O(C₁-C₆)-Alkyl, CO₂H, CONH₂, SO₂NH(C₁-C₆)-Alkyl und SO₂N((C₁-C₆)-Alkyl)₂ ausgewählt ist; und
R⁵ und R⁶ jeweils unabhängig aus Wasserstoff, Cyano und (C₁-C₆)-Alkyl ausgewählt sind;
oder R⁵ und R⁶ zusammen einen 3- bis 6-gliedrigen Cycloalkylring bilden.

2. Verbindung zur Verwendung nach Anspruch 1, wobei m 0 oder 1 ist; n 0, 1 oder 2 ist und p 0 oder 1 ist.

3. Verbindung zur Verwendung nach entweder Anspruch 1 oder Anspruch 2, wobei
R¹ aus Chlor, Fluor, Methyl, Ethyl und Methoxy ausgewählt ist;
R^{1a} aus Wasserstoff, Chlor, Fluor, Cyano, Hydroxy, Methyl, Ethyl, Methoxy und Methoxymethyl ausgewählt ist; und
R^{1b} aus Wasserstoff, Chlor, Fluor, Cyano, Hydroxy, Methyl, Ethyl, Methoxy und Methoxymethyl ausgewählt ist.

4. Verbindung zur Verwendung nach einem der Ansprüche 1 bis 3, wobei:
(i) m 0 ist, R^{1a} Wasserstoff ist und R^{1b} Methyl ist;
(ii) m 1 ist, R¹ Methyl ist und R^{1a} und R^{1b} jeweils Wasserstoff sind; oder
(iii) m 0 ist und R^{1a} und R^{1b} jeweils Wasserstoff sind.

5. Verbindung zur Verwendung nach einem der Ansprüche 1 bis 4, wobei
R² aus Wasserstoff und Methyl ausgewählt ist;
jedes R³ unabhängig aus Wasserstoff, Chlor, Fluor, Cyano, Hydroxy, Methyl, Ethyl, Propyl, Methoxy, Ethoxy und Methoxymethyl ausgewählt ist;
jedes R⁴ unabhängig aus Chlor, Fluor, Cyano, Hydroxy, Methyl, Ethyl, Propyl, Methoxy, Ethoxy, Methoxymethyl, CF³ und OCF³ ausgewählt ist; und
R⁵ und R⁶ jeweils unabhängig aus Wasserstoff, Cyano und Methyl ausgewählt sind.

6. Verbindung zur Verwendung nach Anspruch 1, die ausgewählt ist aus:
1-(3-Cyanophenyl)-N-((1-cyanopyrrolidin-3-yl)methyl)-1H-1,2,4-triazol-3-carboxamid;
(R)-1-(3-Cyanophenyl)-N-((1-cyanopyrrolidin-3-yl)methyl)-1H-1,2,4-triazol-3-carboxamid;
(S)-1-(3-Cyanophenyl)-N-((1-cyanopyrrolidin-3-yl)methyl)-1H-1,2,4-triazol-3-carboxamid;
1-(3-Cyanophenyl)-N-((1-cyanopyrrolidin-3-yl)methyl)-N-methyl-1H-1,2,4-triazol-3-carboxamid;
(R)-1-(3-Cyanophenyl)-N-((1-cyanopyrrolidin-3-yl)methyl)-N-methyl-1H-1,2,4-triazol-3-carboxamid;
(S)-1-(3-Cyanophenyl)-N-((1-cyanopyrrolidin-3-yl)methyl)-N-methyl-1H-1,2,4-triazol-3-carboxamid;
N-((1-Cyanopyrrolidin-3-yl)methyl)-1-phenyl-1H-1,2,4-triazol-3-carboxamid;
(R)-N-((1-Cyanopyrrolidin-3-yl)methyl)-1-phenyl-1H-1,2,4-triazol-3-carboxamid;
(S)-N-((1-Cyanopyrrolidin-3-yl)methyl)-1-phenyl-1H-1,2,4-triazol-3-carboxamid;
N-(((2S,3S)-1-Cyano-2-methylpyrrolidin-3-yl)methyl)-1-(3-cyanophenyl)-1H-1,2,4-triazol-3-carboxamid;
N-(((2S,3R)-1-Cyano-2-methylpyrrolidin-3-yl)methyl)-1-(3-cyanophenyl)-1H-1,2,4-triazol-3-carboxamid;
N-(((2R)-1-Cyano-2-methylpyrrolidin-3-yl)methyl)-1-(3-cyanophenyl)-1H-1,2,4-triazol-3-carboxamid;
N-(((2R,3R)-1-Cyano-2-methylpyrrolidin-3-yl)methyl)-1-(3-cyanophenyl)-1H-1,2,4-triazol-3-carboxamid;
N-(((2R,3S)-1-Cyano-2-methylpyrrolidin-3-yl)methyl)-1-(3-cyanophenyl)-1H-1,2,4-triazol-3-carboxamid;
N-(((2S)-1-Cyano-2-methylpyrrolidin-3-yl)methyl)-1-(3-cyanophenyl)-1H-1,2,4-triazol-3-carboxamid;
1-(3-Cyanophenyl)-N-((1-cyanopyrrolidin-3-yl)methyl)-1H-1,2,3-triazol-4-carboxamid;
(R)-1-(3-Cyanophenyl)-N-((1-cyanopyrrolidin-3-yl)methyl)-1H-1,2,3-triazol-4-carboxamid;
(S)-1-(3-Cyanophenyl)-N-((1-cyanopyrrolidin-3-yl)methyl)-1H-1,2,3-triazol-4-carboxamid;
1-(3-Chlorphenyl)-N-((1-cyanopyrrolidin-3-yl)methyl)-1H-1,2,3-triazol-4-carboxamid;
(R)-1-(3-Chlorphenyl)-N-((1-cyanopyrrolidin-3-yl)methyl)-1H-1,2,3-triazol-4-carboxamid;
(S)-1-(3-Chlorphenyl)-N-((1-cyanopyrrolidin-3-yl)methyl)-1H-1,2,3-triazol-4-carboxamid;
2-(3-Chlorphenyl)-N-((1-cyanopyrrolidin-3-yl)methyl)-2H-1,2,3-triazol-4-carboxamid;
(R)-2-(3-Chlorphenyl)-N-((1-cyanopyrrolidin-3-yl)methyl)-2H-1,2,3-triazol-4-carboxamid;
(S)-2-(3-Chlorphenyl)-N-((1-cyanopyrrolidin-3-yl)methyl)-2H-1,2,3-triazol-4-carboxamid;
N-((1-Cyanopyrrolidin-3-yl)methyl)-2-phenyloxazol-5-carboxamid;
N-((1-Cyanopyrrolidin-3-yl)methyl)-3-phenylisoxazol-5-carboxamid;
N-((1-Cyanopyrrolidin-3-yl)methyl)-5-phenyl-1H-pyrazol-3-carboxamid;
N-((1-Cyanopyrrolidin-3-yl)methyl)-3-(o-tolyl)-1H-pyrazol-5-carboxamid;
N-((1-Cyanopyrrolidin-3-yl)methyl)-2-phenylthiazol-4-carboxamid;
N-((1-Cyanopyrrolidin-3-yl)methyl)-3-(2-fluorphenyl)-1H-pyrazol-5-carboxamid;
N-((1-Cyanopyrrolidin-3-yl)methyl)-5-phenyloxazol-2-carboxamid;
(R)-3-(3-Chlorphenyl)-N-((1-cyanopyrrolidin-3-yl)methyl)isoxazol-5-carboxamid;
(R)-N-((1-Cyanopyrrolidin-3-yl)methyl)-5-phenylosoxazol-3-carboxamid;
(R)-N-((1-Cyanopyrrolidin-3-yl)methyl)-5-phenylthiazol-2-carboxamid;
(R)-N-((1-Cyanopyrrolidin-3-yl)methyl)-4-phenylthiazol-2-carboxamid;
(R)-N-((1-Cyanopyrrolidin-3-yl)methyl)-1-phenyl-1H-pyrazol-3-carboxamid;
(R)-N-((1-Cyanopyrrolidin-3-yl)methyl)-2-phenyl-1H-imidazol-5-carboxamid;
(R)-3-(2-Chlorphenyl)-N-((1-cyanopyrrolidin-3-yl)methyl)isoxazol-5-carboxamid;
(R)-3-(4-Chlorphenyl)-N-((1-cyanopyrrolidin-3-yl)methyl)isoxazol-5-carboxamid;
(R)-5-(3-Cyanophenyl)-N-((1-cyanopyrrolidin-3-yl)methyl)-1,3,4-oxadiazol-2-carboxamid;
(S)-5-(3-Cyanophenyl)-N-((1-cyanopyrrolidin-3-yl)methyl)-1,3,4-oxadiazol-2-carboxamid;
(R)-N-((1-Cyanopyrrolidin-3-yl)methyl)-1-phenyl-1H-imidazol-4-carboxamid;
(R)-1-(3-Cyanophenyl)-N-((1-cyanopyrrolidin-3-yl)methyl)-1H-imidazol-4-carboxamid;
(R)-1-(4-Cyanophenyl)-N-((1-cyanopyrrolidin-3-yl)methyl)-1H-imidazol-4-carboxamid;
(R)-N-((1-Cyanopyrrolidin-3-yl)methyl)-1-(2-methoxyphenyl)-1H-imidazol-4-carboxamid; und
(R)-N-((1-Cyanopyrrolidin-3-yl)methyl)-1-(3-methoxyphenyl)-1H-imidazol-4-carboxamid;
ein Tautomer davon oder ein pharmazeutisch unbedenkliches Salz der Verbindung oder des Tautomers.

7. Verbindung, die ein USP30-Inhibitor ist, oder pharmazeutisch unbedenkliche Zusammensetzung, umfassend die Verbindung, zur Verwendung bei der Behandlung von Fibrose.

8. Verbindung oder Zusammensetzung nach einem der Ansprüche 1 bis 7 zur Verwendung bei der oralen Behandlung von Fibrose.

9. Verbindung oder Zusammensetzung zur Verwendung nach einem der Ansprüche 1 bis 8, wobei die Behandlung prophylaktisch ist.

10. Verbindung oder Zusammensetzung zur Verwendung nach einem der Ansprüche 1 bis 9, wobei die Fibrose aus Fibrose oder einer fibrotischen Störung ausgewählt ist, die mit der Akkumulation von Extrazellularmatrixbestandteilen assoziiert ist, die nach Trauma, Entzündung, Gewebereparatur, immunologischen Reaktionen, Zellhyperplasie und Neoplasie auftritt.

11. Verbindung oder Zusammensetzung zur Verwendung nach Anspruch 10, wobei die Fibrose aus Fibrose oder einer fibrotischen Störung ausgewählt ist, die mit Erkrankungen von wichtigen Organen, fibroproliferativen Störungen und mit Trauma assoziierter Narbenbildung assoziiert ist.

12. Verbindung oder Zusammensetzung zur Verwendung nach Anspruch 11, wobei die Fibrose aus Fibrose oder einer fibrotischen Störung ausgewählt ist, die mit interstitieller Pneumonie, Leberzirrhose, nichtalkoholischer Fettleber, nichtalkoholischer Steatohepatitis, Nierenerkrankung, Herz- oder Gefäßerkrankung, Erkrankungen des Auges, systemischer und lokaler Sklerodermie, Keloiden, hypertrophen Narben, Atherosklerose, Restenose, Dupuytren'scher Kontraktur, chirurgischen Komplikationen, durch Chemotherapeutika induzierter Fibrose, durch Strahlung induzierter Fibrose, Unfallsverletzung und - verbrennungen, retroperitonealer Fibrose und peritonealer Fibrose/peritonealer Narbenbildung assoziiert ist.

13. Verbindung oder Zusammensetzung zur Verwendung nach Anspruch 12, wobei die Fibrose aus Fibrose oder einer fibrotischen Störung ausgewählt ist, die mit Nierenerkrankung assoziiert ist.

14. Verbindung oder Zusammensetzung zur Verwendung nach Anspruch 13, wobei die Fibrose, die mit interstitieller Lungenerkrankung assoziiert ist, aus Sarkoidose, Silikose, Reaktionen auf Arzneimittel, Infektionen, Kollagenosen, rheumatoider Arthritis, systemischer Sklerose, Sklerodermie, Lungenfibrose, idiopathischer Lungenfibrose, gewöhnlicher interstitieller Pneumonitis, interstitieller Pneumonie, kryptogener fibrosierernder Alveolitis, Bronchiolitis obliterans und Bronchiektasie ausgewählt ist.

15. Verbindung oder Zusammensetzung zur Verwendung nach Anspruch 12, wobei die Fibrose, die mit Leberzirrhose assoziiert ist, aus Zirrhose ausgewählt ist, die mit viraler Hepatitis, Schistosomiasis und chronischem Alkoholismus assoziiert ist.

## Revendications

1. Composé de formule (I) : 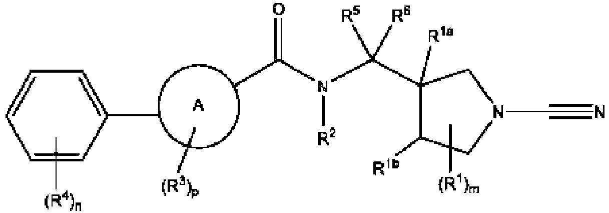 tautomère de celui-ci, ou sel pharmaceutiquement acceptable dudit composé ou tautomère, ou composition pharmaceutiquement acceptable comprenant ledit composé ou sel, pour son utilisation dans le traitement de la fibrose, où :
m va de 0 à 5 ;
n va de 0 à 5 ;
p va de 0 à 3 ;
le cycle A est un cycle hétéroaryle monocyclique contenant 1, 2 ou 3 hétéroatomes, chacun étant indépendamment choisi parmi N, O et S ;
chaque R¹ est indépendamment choisi parmi un groupe halogéno, cyano, hydroxy, (C₁-C₆)alkyle, (C₁-C₆)alcoxy, halogéno(C₁-C₆)alkyle, halogéno(C₁-C₆)alcoxy et (C₁-C₆)alcoxy(C₁-C₆)alkyle ;
R^{1a} et R^{1b} sont chacun indépendamment choisis parmi un groupe hydrogène, halogéno, cyano, hydroxy, (C₁-C₆)alkyle, (C₁-C₆)alcoxy, halogéno(C₁-C₆)alkyle, halogéno(C₁-C₆)alcoxy et (C₁-C₆)alcoxy(C₁-C₆)alkyle ;
R² est choisi parmi un groupe hydrogène, (C₁-C₆)alkyle et (C₁-C₆)alcoxy(C₁-C₆)alkyle ;
chaque R³ est indépendamment choisi parmi un groupe hydrogène, halogéno, cyano, hydroxy, (C₁-C₆)alkyle, (C₁-C₆)alcoxy, (C₁-C₆)alcoxy(C₁-C₆)alkyle, halogéno(C₁-C₆)alkyle, halogéno(C₁-C₆)alcoxy, NH(C₁-C₆)alkyle, N((C₁-C₆)alkyle)₂, C(O)NH(C₁-C₆)alkyle, C(O)N((C₁-C₆)alkyle)₂, NHC(O)(C₁-C₆)alkyle, N((C₁-C₆)alkyl)C(O)(C₁-C₆)alkyle, C(O)(C₁-C₆)alkyle, C(O)O(C₁-C₆)alkyle, CO₂H, CONH₂, SO₂NH(C₁-C₆)alkyle et SO₂N((C₁-C₆)alkyle)₂ ;
chaque R⁴ est indépendamment choisi parmi un groupe halogéno, cyano, hydroxy, (C₁-C₆)alkyle, (C₁-C₆)alcoxy, (C₁-C₆)alcoxy(C₁-C₆)alkyle, halogéno(C₁-C₆)alkyle, halogéno(C₁-C₆)alcoxy, NH(C₁-C₆)alkyle, N((C₁-C₆)alkyle)₂, C(O)NH(C₁-C₆)alkyle, C(O)N((C₁-C₆)alkyle)₂, NHC(O)(C₁-C₆)alkyle, N((C₁-C₆)alkyl)C(O)(C₁-C₆)alkyle, C(O)(C₁-C₆)alkyle, C(O)O(C₁-C₆)alkyle, CO₂H, CONH₂, SO₂NH(C₁-C₆)alkyle et SO₂N((C₁-C₆)alkyle)₂ ; et
R⁵ et R⁶ sont chacun indépendamment choisis parmi un groupe hydrogène, cyano et (C₁-C₆)alkyle ; ou R⁵ et R⁶ forment ensemble un cycle cycloalkyle de 3 à 6 chaînons.

2. Composé pour son utilisation selon la revendication 1, où m vaut 0 ou 1 ; n vaut 0, 1 ou 2 ; et p vaut 0 ou 1.

3. Composé pour son utilisation selon la revendication 1 ou 2, où :
R¹ est choisi parmi un groupe chloro, fluoro, méthyle, éthyle et méthoxy ;
R^{1a} est choisi parmi un groupe hydrogène, chloro, fluoro, cyano, hydroxy, méthyle, éthyle, méthoxy et méthoxyméthyle ; et
R^{1b} est choisi parmi un groupe hydrogène, chloro, fluoro, cyano, hydroxy, méthyle, éthyle, méthoxy et méthoxyméthyle.

4. Composé pour son utilisation selon l'une quelconque des revendications 1 à 3, où :
(i) m vaut 0, R^{1a} est l'hydrogène et R^{1b} est le méthyle ;
(ii) m est 1, R¹ est le méthyle, et R^{1a} et R^{1b} sont chacun l'hydrogène ; ou
(iii) m vaut 0, et R^{1a} et R^{1b} sont chacun l'hydrogène.

5. Composé pour son utilisation selon l'une quelconque des revendications 1 à 4, où :
R² est choisi parmi l'hydrogène et le méthyle ;
chaque R³ est choisi indépendamment parmi un groupe hydrogène, chloro, fluoro, cyano, hydroxy, méthyle, éthyle, propyle, méthoxy, éthoxy et méthoxyméthyle ;
chaque R⁴ est choisi indépendamment parmi un groupe chloro, fluoro, cyano, hydroxy, méthyle, éthyle, propyle, méthoxy, éthoxy, méthoxyméthyle, CF₃ et OCF₃ ; et
R⁵ et R⁶ sont chacun indépendamment choisis parmi un groupe hydrogène, cyano et méthyle.

6. Composé pour son utilisation selon la revendication 1, qui est choisi parmi un groupe :
1-(3-cyanophényl)-N-((1-cyanopyrrolidin-3-yl)méthyl)-1H-1,2,4-triazole-3-carboxamide ;
(R)-1-(3-cyanophényl)-N-((1-cyanopyrrolidin-3-yl)méthyl)-1H-1,2,4-triazole-3-carboxamide ;
(S)-1-(3-cyanophényl)-N-((1-cyanopyrrolidin-3-yl)méthyl)-1H-1,2,4-triazole-3-carboxamide ;
1-(3-cyanophényl)-N-((1-cyanopyrrolidin-3-yl)méthyl)-N-méthyl-1H-1,2,4-triazole-3-carboxamide ;
(R)-1-(3-cyanophényl)-N-((1-cyanopyrrolidin-3-yl)méthyl)-N-méthyl-1H-1,2,4-triazole-3-carboxamide ;
(S)-1-(3-cyanophényl)-N-((1-cyanopyrrolidin-3-yl)méthyl)-N-méthyl-1H-1,2,4-triazole-3-carboxamide ;
N-((1-cyanopyrrolidin-3-yl)méthyl)-1-phényl-1H-1,2,4-triazole-3-carboxamide ;
(R)-N-((1-cyanopyrrolidin-3-yl)méthyl)-1-phényl-1H-1,2,4-triazole-3-carboxamide ;
(S)-N-((1-cyanopyrrolidin-3-yl)méthyl)-1-phényl-1H-1,2,4-triazole-3-carboxamide ;
N-(((2S,3S)-1-cyano-2-méthylpyrrolidin-3-yl)méthyl)-1-(3-cyanophényl)-1H-1,2,4-triazole-3-carboxamide ;
N-(((2S,3R)-1-cyano-2-méthylpyrrolidin-3-yl)méthyl)-1-(3-cyanophényl)-1H-1,2,4-triazole-3-carboxamide ;
N-(((2S,3R)-1-cyano-2-méthylpyrrolidin-3-yl)méthyl)-1-(3-cyanophényl)-1H-1,2,4-triazole-3-carboxamide ;
N-(((2R,3R)-1-cyano-2-méthylpyrrolidin-3-yl)méthyl)-1-(3-cyanophényl)-1H-1,2,4-triazole-3-carboxamide ;
N-(((2R,3S)-1-cyano-2-méthylpyrrolidin-3-yl)méthyl)-1-(3-cyanophényl)-1H-1,2,4-triazole-3-carboxamide ;
N-(((2S)-1-cyano-2-méthylpyrrolidin-3-yl)méthyl)-1-(3-cyanophényl)-1H-1,2,4-triazole-3-carboxamide ;
1-(3-cyanophényl)-N-((1-cyanopyrrolidin-3-yl)méthyl)-1H-1,2,3-triazole-4-carboxamide ;
(R)-1-(3-cyanophényl)-N-((1-cyanopyrrolidin-3-yl)méthyl)-1H-1,2,3-triazole-4-carboxamide ;
(S)-1-(3-cyanophényl)-N-((1-cyanopyrrolidin-3-yl)méthyl)-1H-1,2,3-triazole-4-carboxamide ;
1-(3-chlorophényl)-N-((1-cyanopyrrolidin-3-yl)méthyl)-1H-1,2,3-triazole-4-carboxamide ;
(R)-1-(3-chlorophényl)-N-((1-cyanopyrrolidin-3-yl)méthyl)-1H-1,2,3-triazole-4-carboxamide ;
(S)-1-(3-chlorophényl)-N-((1-cyanopyrrolidin-3-yl)méthyl)-1H-1,2,3-triazole-4-carboxamide ;
2-(3-chlorophényl)-N-((1-cyanopyrrolidin-3-yl)méthyl)-2H-1,2,3-triazole-4-carboxamide ;
(R)-2-(3-chlorophényl)-N-((1-cyanopyrrolidin-3-yl)méthyl)-2H-1,2,3-triazole-4-carboxamide ;
(S)-2-(3-chlorophényl)-N-((1-cyanopyrrolidin-3-yl)méthyl)-2H-1,2,3-triazole-4-carboxamide ;
N-((1-cyanopyrrolidin-3-yl)méthyl)-2-phényloxazole-5-carboxamide ;
N-((1-cyanopyrrolidin-3-yl)méthyl)-3-phénylisoxazole-5-carboxamide ;
N-((1-cyanopyrrolidin-3-yl)méthyl)-5-phényl-1H-pyrazole-3-carboxamide ;
N-((1-cyanopyrrolidin-3-yl)méthyl)-3-(o-tolyl)-1H-pyrazole-5-carboxamide ;
N-((1-cyanopyrrolidin-3-yl)méthyl)-2-phénylthiazole-4-carboxamide ;
N-((1-cyanopyrrolidin-3-yl)méthyl)-3-(2-fluorophényl)-1H-pyrazole-5-carboxamide ;
N-((1-cyanopyrrolidin-3-yl)méthyl)-5-phényloxazole-2-carboxamide ;
(R)-3-(3-chlorophényl)-N-((1-cyanopyrrolidin-3-yl)méthyl)isoxazole-5-carboxamide ;
(R)-N-((1-cyanopyrrolidin-3-yl)méthyl)-5-phénylisoxazole-3-carboxamide ;
(R)-N-((1-cyanopyrrolidin-3-yl)méthyl)-5-phénylthiazole-2-carboxamide ;
(R)-N-((1-cyanopyrrolidin-3-yl)méthyl)-4-phénylthiazole-2-carboxamide ;
(R)-N-((1-cyanopyrrolidin-3-yl)méthyl)-1-phényl-1H-pyrazole-3-carboxamide ;
(R)-N-((1-cyanopyrrolidin-3-yl)méthyl)-2-phényl-1H-imidazole-5-carboxamide ;
(R)-3-(2-chlorophényl)-N-((1-cyanopyrrolidin-3-yl)méthyl)isoxazole-5-carboxamide ;
(R)-3-(4-chlorophényl)-N-((1-cyanopyrrolidin-3-yl)méthyl)isoxazole-5-carboxamide ;
(R)-5-(3-cyanophényl)-N-((1-cyanopyrrolidin-3-yl)méthyl)-1,3,4-oxadiazole-2-carboxamide ;
(S)-5-(3-cyanophényl)-N-((1-cyanopyrrolidin-3-yl)méthyl)-1,3,4-oxadiazole-2-carboxamide ;
(R)-N-((1-cyanopyrrolidin-3-yl)méthyl)-1-phényl-1H-imidazole-4-carboxamide ;
(R)-1-(3-cyanophényl)-N-((1-cyanopyrrolidin-3-yl)méthyl)-1H-imidazole-4-carboxamide ;
(R)-1-(4-cyanophényl)-N-((1-cyanopyrrolidin-3-yl)méthyl)-1H-imidazole-4-carboxamide ;
(R)-N-((1-cyanopyrrolidin-3-yl)méthyl)-1-(2-méthoxophényl)-1H-imidazole-4-carboxamide ; et
(R)-N-((1-cyanopyrrolidin-3-yl)méthyl)-1-(3-méthoxophényl)-1H-imidazole-4-carboxamide ;
tautomère de celui-ci, ou sel pharmaceutiquement acceptable dudit composé ou tautomère.

7. Composé qui est un inhibiteur de USP30, ou composition pharmaceutiquement acceptable comprenant ledit composé, pour son utilisation dans le traitement de la fibrose.

8. Composé ou composition selon l'une quelconque des revendications 1 à 7 pour son utilisation dans le traitement oral de la fibrose.

9. Composé ou composition pour son utilisation selon l'une quelconque des revendications 1 à 8, le traitement étant prophylactique.

10. Composé ou composition pour son utilisation selon l'une quelconque des revendications 1 à 9, la fibrose étant choisie parmi une fibrose ou un trouble fibrotique associé à l'accumulation de constituants de la matrice extracellulaire qui survient à la suite d'un traumatisme, d'une inflammation, d'une réparation tissulaire, de réactions immunologiques, d'une hyperplasie cellulaire et d'une néoplasie.

11. Composé ou composition pour son utilisation selon la revendication 10, la fibrose étant choisie parmi une fibrose ou un trouble fibrotique associé à des maladies d'organes majeurs, des troubles fibroprolifératifs et une cicatrisation associée à un traumatisme.

12. Composé ou composition pour son utilisation selon la revendication 11, la fibrose étant choisie parmi une fibrose ou un trouble fibrotique associé à une maladie pulmonaire interstitielle, une cirrhose du foie, une maladie du foie gras non alcoolique, une stéatohépatite non alcoolique, une maladie rénale, une maladie cardiaque ou vasculaire, des maladies de l'œil, une sclérodermie systémique et locale, des chéloïdes, des cicatrices hypertrophiques, l'athérosclérose, une resténose, la maladie de Dupuytren, des complications chirurgicales, une fibrose induite par un médicament chimiothérapeutique, une fibrose induite par des radiations, une lésion accidentelle et des brûlures, une fibrose rétropéritonéale et une fibrose péritonéale/cicatrisation péritonéale.

13. Composé ou composition pour son utilisation selon la revendication 12, la fibrose étant choisie parmi une fibrose ou un trouble fibrotique associé à une maladie rénale.

14. Composé ou composition pour son utilisation selon la revendication 13, la fibrose associée à une maladie pulmonaire interstitielle étant choisie parmi une sarcoïdose, une silicose, des réactions médicamenteuses, des infections, des maladies vasculaires du collagène, la polyarthrite rhumatoïde, une sclérose systémique, une sclérodermie, une fibrose pulmonaire, la fibrose pulmonaire idiopathique, la pneumopathie interstitielle commune, une maladie pulmonaire interstitielle, l'alvéolite fibrosante cryptogénique, la bronchiolite oblitérante et une bronchectasie.

15. Composé ou composition pour son utilisation selon la revendication 12, la fibrose associée à une cirrhose du foie étant choisie parmi une cirrhose associée à une hépatite virale, une schistosomiase et l'alcoolisme chronique.
